# EUROPEAN PATENT APPLICATION

(11) **EP 4 400 518 A1**
(43) Date of publication of application: **17.07.2024**
(21) Application number: 22866731.7
(22) Date of filing: 08.09.2022
(51) Int. Cl.: C07K 19/00, A61K 39/395, A61P 37/02, A61P 35/00

(54) **ANTIGEN-TARGETING, ANTI-CD16A AND IMMUNE EFFECTOR CELL-ACTIVATING TRIFUNCTIONAL FUSION PROTEIN, AND USE THEREOF**

(30) Priority: 09.09.2021 CN 202111064677
(71) Applicant: Qure Biotechnology (Shanghai) Co., Ltd., Shanghai 201203 (CN)
(72) Inventor: QU, Xiangdong, Shanghai 201203 (CN); PAN, Qin, Shanghai 201203 (CN)
(74) Representative: Cabinet Laurent & Charras
(86) International application number: PCT/CN2022/117940
(87) International publication number: WO 2023/036270

(57) **Abstract**

Disclosed is an antigen-targeting, anti-CD16A and immune effector cell-activating trifunctional fusion protein, which comprises a CD16A binding region that specifically binds to CD16A, a TAA binding region that specifically binds to a tumor-associated antigen (TAA), an II,-15/II,-15 Rα complex formed by means of binding of IL-15 to IL-15 Rα, and an FC domain. The designed tumor-targeting innate immune cell activating molecule, i.e. the anti-TAA, anti-CD 16A and cytokine ILl5 trifunctional fusion protein, can simultaneously bind to a TAA on a tumor cell and CD16A on an NK cell, thereby making the NK cell target tumors. The NK cell releases perforin and granzyme to induce apoptosis and cause the death of the tumor cells. The cytokine IL 15 is expanded to maintain the function of the NK cell, stimulate the proliferation of immune cells, and change the immune microenviroument of the tumor.

## Description

### TECHNICAL FIELD

The present invention relates to the field of biopharmaceutical technology, in particular to an antigen targeting, anti-CD16A, and immune effector cell activating trifunctional fusion protein, and application thereof.

### BACKGROUND

Natural killer cells (NK cells) are a type of natural immune cells that can effectively destroy stress cells, such as virus infected or tumor transformed cells. NK cells have functions such as cytotoxicity, cytokine production, and immune memory that are not limited by MHC, making them a key role in the immune response system. Antibody dependent cell-mediated cytotoxicity (ADCC) is an effective cytotoxic mechanism primarily mediated by natural killer (NK) cells. The ADCC effect by targeting CD16a is one of the main mechanisms underlying the anti-tumor effect of NK cells. When specific antibodies bind to the targeted tumor cell membrane, immune cells induce cell death through this reaction.

The interaction between antibodies and immune cells occurs through the Fc receptor (FcR) family. In humans, the FcR family of IgG (Fc y R) is composed of 6 receptors: Fc y RI/CD64, Fc y RIIa/CD32a, Fc y RIIb/CD32b, Fc y RIIc/CD32c, Fc y RIIIa/CD16a and Fc y RIIIb/CD16b, among which CD16a mainly triggers NK cell-mediated ADCC. Usually, the ADCC effect is mainly achieved by activating NK cells by antibodies binding to Fc y RIII (CD16). Human NK cells are divided into two main subgroups: CD56 bright and CD56 dim. CD56 bright NK cells are powerful cytokine producers, but lack CD16a; CD56 dim has high cytotoxicity and expresses CD16a. When CD16a recognizes the target of IgG regulation, NK cells release different cytotoxic molecules, leading to the death of target cells. This mechanism relies on the formation of immune synapses and the degranulation of dissolved particles containing perforin and granzymes. In addition to degranulation, NK cells can also clear target cells by binding target death receptors (such as DR4, DR5, or Fas) to their death receptor ligands (such as FasL and TRAIL).

CD16a is a transmembrane receptor with a short C-ter cytoplasmic tail and two extracellular Ig like domains. CD16a interacts with the CH2 of IgG in a 1:1 manner, with the N-glycan chain at the N297 position on CH2 also playing a crucial role in this interaction. CD16a does not have an ITAM domain at its cytoplasmic tail, so it requires the assistance of two intracellular chains containing ITAM domains in tandem, CD3 ζ and Fc ε RI y. After CD16a binding, the kinase Lck belonging to the Src family is activated and phosphorylates the ITAM domain of CD3 ζ and/or FcεRIγ. Phosphorylated ITAM allows for recruitment and phosphorylation of kinases from the Syk family, such as Syk and ZAP-70, which in turn are responsible for subsequent signaling. Among their substrates, PI3K is highly correlated as it converts PIP2 to PIP3, which is then processed by PLC-y to release IP3 and DAG. DAG activates the PKC family, which helps trigger degranulation. IP3 induces calcium release from the endoplasmic reticulum, and this calcium influx is one of the main signals triggered by ADCC. It also leads to NFAT translocation in the nucleus, inducing transcription of its target genes. Other pathways involved in CD16a activation also contribute to ADCC, such as the ERK2 MAPK pathway. After CD16a dependent NK cell killing, CD16a is rapidly downregulated. One of the mechanisms underlying the shedding of CD16a is mediated by a disintegrin and metalloproteinase domain 17 (ADAM17), which is expressed on NK cells. The cleavage of CD16a by ADAM17 occurs in the cis structure, which means that a NK cell expressing ADAM17 cannot induce CD16a shedding on another NK cell. Another mechanism for the downregulation of CD16a after activation is internalization, which occurs not only on CD16a but also on other intracellular signaling components, such as CD3 ζ, ZAP-70 and Syk.

NK cells, as killer cells in innate immune cells, can directly kill pathogens and tumor cells. Compared with T cells, NK cells have stronger tumor recognition and killing abilities, as well as stronger potential for spot application. How to modify the constant region (Fc end) of therapeutic antibody drugs to better activate innate immune responses, enhance antibody dependent cell mediated cytotoxicity (ADCC), and evaluate the dual (or multiple) anti-drug efficacy that can specifically bind to CD16A are issues of interest to drug development scientists. The current strategies to improve the effectiveness of ADCC mainly include the following aspects:
(1) Cytokines: A simple way to enhance ADCC activity is to stimulate NK cells with pro-inflammatory cytokines. NK cell transplantation has been successfully performed in several clinical trials, such as by infusing cytokine induced memory like (CIML) NK cells, with the most used cytokine combinations including IL-12, IL-15, and IL-18. CIML NK cells express more IFN- y than traditional NK cells, and exhibit superior cytotoxicity to leukemia cells and primary acute myeloid leukemia (AML) blastoid cells. IL-12 increases the production of IFN-γ and TNF-α by NK cells. In a phase I/II clinical trial, cetuximab was used in combination with IL-12 in patients with unresectable or recurrent head and neck squamous cell carcinoma. Compared with patients with progression free survival (PFS) less than 100 days, patients with PFS greater than 100 days had higher ADCC activity *in vitro.* IL-15 is a necessary cytokine for the survival and function of NK and CD8+T cells. N-803 is a mutated N72D IL-15 super agonist that has shown enhanced *in vitro* and *in vivo* activity of NK cells in preclinical models and clinical trials. Furthermore, it also increased the number of NK and CD8+T cells.
(2) Inhibition of ADAM17: ADAM17 is the main driving factor for the downregulation of CD16a after activation. One strategy to improve ADCC of NK cells is to prevent CD16a shedding by targeting ADAM17. Researches have found that knocking out ADAM17 using CRISPR/Cas9 technology in NK cells displayed better IFN-γ generation and *in vivo* and *in vitro* ADCC activity. However, some studies have also shown that blocking ADAM17 reduced the survival rate of NK cells and CD16a mediated serial killing, prevented cells from separating from target cells and hindered their movement to another target cell. In summary, the benefits of ADAM17 inhibition are not yet clear, and the results are also controversial. The success of this strategy depends on the results of an ongoing clinical trial (NCT04023071), which will evaluate the application of iPSC-derived NK cells carrying non-cleavable CD16a in AML and B-cell lymphoma.
(3) Engineering antibody: The Fc part of an antibody can be engineered to increase its affinity for CD16a and subsequently improve ADCC. Some mutations are very prevalent, such as the so-called GASDALIE, which consists of four substitutions in Fc: G236A/S239D/A330L/I332E. This mutation showed a significant increase in affinity for CD16a, while the affinity for CD32b remained almost unchanged. Another mutation is called mutant 18 (F243L/R292P/Y300L/V305I/P396L), with a significant increase in ADCC. These mutations have now entered clinical practice as the anti-HER2 antibody margetuximab and have shown good efficacy in the treatment of various HER2 positive cancers in Phase I. In addition, the glycosy engineering of antibody Fc N-glycan is expected to increase its affinity for CD16a. The most extensively studied glycosy modification is deglycosylation, such as de fucose modification. This modification leads to increased binding with CD16a and improved ADCC. This technology has been used for anti-CD20 antibody obinuzumab.
(4) NK cell engager: In addition to engineered antibodies, the form of antibodies can be studied. Based on the success of the bispecific T cell engager (BiTE) strategy, a similar form has been developed for NK cells, known as the bispecific killer cell engager (BiKE). BiKE is composed of two scFv linked sequences, one single chain antibody targeting CD16a and the other targeting tumor antigen. In addition, there is a triple specific killer cell engager (TRiKE), which targets two tumor antigens together with CD16a, or added with IL-15 instead of the third single chain antibody. The use of this structure allows NK cells to relocate to tumor cells and leads to strong ADCC effects against target cells. AFM13 is a bispecific antibody targeting CD16a and CD30, currently in clinical Phase II, which is used for the treatment of peripheral T-cell lymphoma, transformed mycosis fungoides, and Hodgkin's lymphoma. In a Phase Ib clinical study for the treatment of recurrent or refractory Hodgkin's lymphoma, the combination of AFM13 and pembrolizumab resulted in an objective response rate of 88%, with a total response rate of 83%. GTB-3550 is a TRiKE form of CD16/CD33/IL-15, currently undergoing phase I/II trials for various types of leukemia (NCT03214666).

However, in most tumors, including colon cancer, kidney cancer, liver cancer, breast cancer and lung cancer, the number of NK cells in the tumor microenvironment is very small. Therefore, the industry has developed an immunotherapy method that utilizes the activity of CD16A to treat cancer, such as bispecific molecules formed by antibodies specific to tumor surface antigens and NK cell CD16A. Currently, bispecific antibodies for HER2/neu and CD16A have been developed, which have stronger anti-tumor activity than HER2 antibodies alone; AFM13, a bispecific antibody that specifically binds to CD16A and CD30, by binding to CD16 molecules on the surface of NK cells or monocytes, allows these immune effector cells to target CD30 expressing tumor cells for killing. This antibody has shown tumor clearance effects in both Hodgkin's tumor mouse models and patients. However, exposure to traditional CD16A antibodies or anti-CD16A antigen binding proteins for a period of time can lead to NK cell failure or reduced cytotoxicity, thereby affecting the tumor killing effect of NK cells. It results in the inability of CD16A dual antibody to be administered for a long time, which affects its effectiveness.

Interleukin-15 (IL-15) is a 14-15 kDa cytokine that is crucial for the function of NK cells, NK T cells, and memory CD8+T cells. IL-15 binds to its receptor IL-15Rα to produce a highly biologically potent complex, IL-15 superagonist (IL-15 SA), which is then transduced and transported to target cells. IL-15 SA strongly activates cells expressing IL-15R, especially NK cells/T cells, thereby promoting anti-tumor and antiviral functions. IL-15 has a wide range of immune regulatory effects and can participate in regulating the survival, proliferation, and function of T cells, especially NK cells and memory CD8+T cells. IL-15 and IL-2 have very similar structures and belong to the spiral cytokine family. The heterotrimeric receptor of IL-15 shares IL-2R/IL-15 Rβ (CD122) and common y c-chain (CD 132) with IL-2 receptor, due to its shared receptor components and shared JAK 1/JAK 3/STAT 5 signaling pathway, IL-15 shares the same functions as IL-2, including stimulating T cell proliferation, producing cytotoxic T lymphocytes, stimulating synthesis of immunoglobulin by B cells, and the production and sustained survival of NK cells. In many adaptive immune responses, IL-2 and IL-15 play a unique and often competitive role. There are four main points: 1. IL-2 can regulate the activation of Tregs cells, while IL-15 cannot. 2. IL-2 can inhibit T cell response by activating induced CD8+effector T cell death. 3. IL-15 plays an essential role in the differentiation of NK, effector CD8+cells, and memory phenotype CD8+T cells. 4. Preclinical studies have shown that the toxicity of IL-15 is different from that of IL-2, and compared to IL-2, IL-15 shows almost no vascular capillary leakage. These factors make IL-15 a more promising cytokine in tumor immune strategies. At present, several IL-15 target products are still in clinical trials, among which the most noteworthy is IL-15 super agonist N-803, which is a fusion protein co-expressed by IL-15 protein N72D mutation combined with IL-15Ra and IgGlFc. On May 23, 2022, ImmunityBio submitted the marketing application of IL-15 super agonist N-803 to the FDA for combined treatment of non-muscle invasive bladder cancer (NMIBC) that does not respond to BCG with BCG. The clinical data released in 2021 showed that the combination therapy of N-803+BCG had a significant effect, with a CR rate of 71% (59/83) and an average CR maintenance time of 24.1 months. A phase I clinical trial showed that the combination of Nivolumab (PD-1 antibody, Opdivo) and N-803 in the treatment of metastatic non-small cell lung cancer could significantly prolong the long-term survival of patients. The IL-15 cytokine XmAb^{®}24306, which is co-developed by Genentech and Xencor, currently is undergoing XmAb^{®}24306 combined with Tecntriq clinical study. SHR-1501 (IL-15 fusion protein) from Hengrui Pharmaceutical was approved for clinical trials on May 14, 2019. Moreover, SHR-1316 (PD-L1 monoclonal antibody drug) will be used in combination with SHR-1501 for the treatment of advanced malignant tumor patients who have failed in therapy. However, the clinical use of cytokines has the disadvantage of poor single drug targeting. Only high concentration administration can achieve anti-tumor effects, while high concentration administration can produce immunosuppressive effects and high toxicity. Moreover, the activation of the immune system by non-targeted cytokines is systemic, and the immune system is widely activated, with fatal side effects. In addition, due to the fact that cytokines belong to small molecular weight proteins and do not have the *in vivo* circulating protection mechanism of antibodies. Simple cytokines often have a short half-life and require short-term repeated high-dose administration. At present, clinical research drugs often use PEGylation or Fc fusion to increase the half-life of cytokines. Although the half-life can be extended, it still cannot solve the problem of poor targeting of cytokines.

### SUMMARY OF THE INVENTION

The first objective of the present invention is to provide an antigen targeting, anti-CD16A, and immune effector cell activating trifunctional fusion protein.

The antigen targeting, anti-CD16A, and immune effector cell activating trifunctional fusion protein provided by the present invention comprises: a CD16A binding region specifically binding to CD16A, a TAA binding region specifically binding to tumor associated antigen (TAA), an IL-15/IL-15Rα complex formed by interaction of IL-15 and IL-15Rα, and a Fc domain. The antigen comprises tumor associated antigen (TAA). The trifunctional fusion protein provided by the present invention not only targets CD16A and TAA simultaneously, but also fuses an IL-15/IL-15Rα complex and a Fc domain. It has unique components and forms a unique configuration. Furthermore, the trifunctional fusion protein of the present invention utilizes IL-15 and IL-15Rα to replace the CL and CH1 domains in one of the antibody structure of a bispecific antibody respectively, by using the IL-15/IL-15Rα structure formed by the ultra-high affinity between IL-15 and IL-15Rα (with a KD value about 30-100pM), to ensure correct pairing between the light and heavy chains of the bispecific antibody, thereby solving the problem of light chain mismatch in the bispecific antibody. At the same time, the CD16A antibody of the trifunctional fusion protein targets NK cells to the tumor site and activates NK cells near the tumor cells. Exposure to traditional CD16A antibodies or anti-CD16A antigen binding proteins for a period of time can lead to NK cell failure or reduced cytotoxicity, resulting in the inability of CD16A antibodies to be administered for a long time and affecting the effectiveness of tumor treatment. However, the damaged NK cell toxicity can be restored by the stimulation of the cytokines IL-15 and IL-15Rα in the trifunctional fusion protein structure of the present invention, thereby enhancing the tumor cell killing effect of NK cells, solving the problem of NK cell depletion and reduced tumor cell toxicity caused by CD16A antibodies. IL-15 and IL-15Rα stimulate the proliferation and activation of immune effector cells such as NK cells and T cells, and greatly enhances the body's immunosuppressive activity against tumors.

Optionally, the CD16A binding region comprises an anti-CD16A antibody and fragment thereof, preferably, the CD16A binding region is a Fab fragment or Fv fragment targeting CD16A.

Optionally, the TAA binding region comprises an anti-TAA antibody and fragment thereof, preferably, the TAA binding region is a Fab fragment or Fv fragment targeting TAA.

Optionally, the fusion protein further comprises a connecting fragment; and the amino acid sequence of the connecting fragment is preferably composed of several GS repeat sequences.

Optionally, the fusion protein comprises several peptide chains, wherein the IL-15 and IL-15Rα are located on different peptide chains, respectively.

Optionally, the trifunctional fusion protein comprises: a described TAA binding region.

Optionally, the fusion protein comprises: a first monomer and a second monomer; wherein, the first monomer comprises: the TAA binding region and a first Fc chain; the second monomer comprises: the CD16A binding region, the IL-15/IL-15Rα complex and a second Fc chain; the TAA binding region is a Fab fragment targeting TAA; the CD16A binding region is a Fv fragment targeting CD16A; or the first monomer comprises: the CD16A binding region and a first Fc chain; the second monomer comprises: the TAA binding region, the IL-15/IL-15Rα complex and a second Fc chain; the CD16A binding region is a Fab fragment targeting CD16A; the TAA binding region is a Fv fragment targeting TAA; and the first Fc chain and the second Fc chain polymerize to form the Fc domain.

Optionally, the first monomer comprises a first peptide chain and a second peptide chain, and the second monomer comprises a third peptide chain and a fourth peptide chain; the first peptide chain comprises: a TAA binding antibody variable region and an antibody constant region; the second peptide chain comprises: a TAA binding antibody variable region and an antibody constant region; the antibody constant region of the second peptide chain comprises a first Fc chain; the third peptide chain comprises: a CD16A binding antibody variable region, a cytokine, and a second Fc chain; the fourth peptide chain comprises: a CD16A binding antibody variable region and a cytokine; the pairing regions of the TAA binding antibody variable region and antibody constant region of the first peptide chain, as well as the TAA binding antibody variable region and antibody constant region of the second peptide chain form the Fab fragment targeting TAA; the CD16A binding antibody variable region of the third peptide chain and the CD16A binding antibody variable region of the fourth peptide chain pair to form the Fv fragment targeting CD16A; the combination of the cytokine of the third peptide chain and the cytokine of the fourth peptide chain forms the IL-15/IL-15Rα complex.

Optionally, the first monomer comprises a first peptide chain and a second peptide chain, and the second monomer comprises a third peptide chain and a fourth peptide chain; the first peptide chain comprises: a CD16A binding antibody variable region and an antibody constant region; the second peptide chain comprises: a CD16A binding antibody variable region and an antibody constant region; the antibody constant region of the second peptide chain comprises a first Fc chain; the third peptide chain comprises: a TAA binding antibody variable region, a cytokine, and a second Fc chain; the fourth peptide chain comprises: a TAA binding antibody variable region and a cytokine; the pairing regions of the CD 16A binding antibody variable region and antibody constant region of the first peptide chain, as well as the CD16A binding antibody variable region and antibody constant region of the second peptide chain form the Fab fragment targeting CD16A; the TAA binding antibody variable region of the third peptide chain and the TAA binding antibody variable region of the fourth peptide chain pair form the Fv fragment targeting TAA; the combination of the cytokine of the third peptide chain and the cytokine of the fourth peptide chain forms the IL-15/IL-15Rα complex.

Optionally, from the N-terminus to the C-terminus of the peptide chain, the fusion order of the third peptide chain is antibody variable region~cytokine~the second Fc chain, or cytokine-antibody variable region-the second Fc chain; the fusion order of the fourth peptide chain from the N-terminus to the C-terminus of the peptide chain is antibody variable region-cytokine; or cytokine-antibody variable region; "~" represents a connecting fragment; the antibody variable region is a CD16A binding antibody variable region or a TAA binding antibody variable region.

Optionally, the trifunctional fusion protein comprises: two described TAA binding regions.

Optionally, the fusion protein comprises: a first monomer and a second monomer; the two described TAA binding regions are the first TAA binding region and the second TAA binding region; wherein, the first monomer comprises: the first TAA binding region and a first Fc chain; the second monomer comprises: the CD16A binding region, the second TAA binding region, the IL-15/IL-15Rα complex and a second Fc chain; the first TAA binding region is a Fab fragment targeting TAA; the CD16A binding region is a Fv fragment targeting CD16A; the second TAA binding region is a Fv fragment targeting TAA; or the first monomer comprises: the first TAA binding region, the CD16A binding region and a first Fc chain; the second monomer comprises: the second TAA binding region, the IL-15/IL-15Rα complex and a second Fc chain; the CD16A binding region is a Fv fragment targeting CD16A; both the first TAA binding region and the second TAA binding region are Fv fragments targeting TAA; and the first Fc chain and the second Fc chain polymerize to form the Fc domain.

Optionally, the first monomer comprises a first peptide chain and a second peptide chain, and the second monomer comprises a third peptide chain and a fourth peptide chain; the first peptide chain comprises: a TAA binding antibody variable region and an antibody constant region; the second peptide chain comprises: a TAA binding antibody variable region and an antibody constant region; the antibody constant region of the second peptide chain comprises a first Fc chain; the third peptide chain comprises: a TAA binding antibody variable region, a CD16A binding antibody variable region, a cytokine, and a second Fc chain; the fourth peptide chain comprises: a TAA binding antibody variable region, a CD 16A binding antibody variable region and a cytokine; the pairing regions of the TAA binding antibody variable region and antibody constant region of the first peptide chain, as well as the TAA binding antibody variable region and antibody constant region of the second peptide chain form the first TAA binding region, and the first TAA binding region is a Fab fragment targeting TAA; the TAA binding antibody variable region of the third peptide chain and the TAA binding antibody variable region of the fourth peptide chain pair form the second TAA binding region, and the second TAA binding region is a Fv fragment targeting TAA; the CD16A binding antibody variable region of the third peptide chain and the CD16A binding antibody variable region of the fourth peptide chain pair to form the CD16A binding region, and the CD16A binding region is a Fv fragment targeting CD16A; the combination of the cytokine of the third peptide chain and the cytokine of the fourth peptide chain forms the IL-15/IL-15Rα complex.

Optionally, from the N-terminus to the C-terminus of the peptide chain, the fusion order of the amino acid fragments of the third peptide chain or the fourth peptide chain comprises any one of the followings; wherein, "∼" represents a connecting fragment:
(1) TAA binding antibody variable region~CD16A binding antibody variable region-cytokine;
(2) TAA binding antibody variable region~cytokine~CD16A binding antibody variable region;
(3) CD16A binding antibody variable region~TAA binding antibody variable region-cytokine;
(4) CD16A binding antibody variable region~cytokine~TAA binding antibody variable region;
(5) cytokine~TAA binding antibody variable region~CD16A binding antibody variable region;
(6) cytokine~CD 16A binding antibody variable region~TAA binding antibody variable region.

Optionally, the fusion protein comprises: a first monomer and a second monomer; wherein the first peptide chain comprises: a TAA binding antibody variable region, a CD16A binding antibody variable region and an antibody constant region; the second peptide chain comprises: a TAA binding antibody variable region, a CD16A binding antibody variable region and an antibody constant region; the antibody constant region of the second peptide chain comprises a first Fc chain; the third peptide chain comprises: a TAA binding antibody variable region, a cytokine, and a second Fc chain; the fourth peptide chain comprises: a TAA binding antibody variable region and a cytokine; the TAA binding antibody variable region of the first peptide chain, as well as the TAA binding antibody variable region of the second peptide chain pair to form the first TAA binding region, and the first TAA binding region is a Fv fragment targeting TAA; the CD16A binding antibody variable region of the first peptide chain and the CD16A binding antibody variable region of the second peptide chain pair to form the CD16A binding region, and the CD16A binding region is a Fv fragment targeting CD 16A; the TAA binding antibody variable region of the third peptide chain and the TAA binding antibody variable region of the fourth peptide chain pair form the second TAA binding region, and the second TAA binding region is a Fv fragment targeting TAA; the combination of the cytokine of the third peptide chain and the cytokine of the fourth peptide chain forms the IL-15/IL-15Rα complex.

Optionally, in the first peptide chain or the second peptide, from the N-terminus to the C-terminus of the peptide chain, the fusion order of the TAA binding antibody variable region and the CD16A binding antibody variable region is: TAA binding antibody variable region~CD 16A binding antibody variable region, or CD16A binding antibody variable region~TAA binding antibody variable region; "~" represents a connecting fragment; or in the third peptide chain or the fourth peptide chain, from the N-terminus to the C-terminus of the peptide chain, the fusion order of the TAA binding antibody variable region and the cytokine is: TAA binding antibody variable region-cytokine, or cytokine~TAA binding antibody variable region.

Optionally, the TAA binding antibody variable region is selected from the VH or VL domain targeting TAA, the CD16A binding antibody variable region is selected from the VH or VL domain targeting CD16A, and the cytokine is selected from IL-15 or IL-15Rα; the antibody constant region comprises a CL domain or a CH1 domain.

Optionally, there are one or more pairs of disulfide bonds between the VH domain and VL domain, and they comprise any one or more of the following mutation combinations, counted according to EU numbering:

| | **Disulfide bond modification sites** | |
|---|---|---|
| | VH | VL |
| Combination 1 | 37C | 95C |
| Combination 2 | 44C | 100C |
| Combination 3 | 44C | 105C |
| Combination 4 | 45C | 87C |
| Combination 5 | 100C | 50C |
| Combination 6 | 100bC | 49C |
| Combination 7 | 98C | 46C |
| Combination 8 | 101C | 46C |
| Combination 9 | 105C | 43C |
| Combination | 106C | 57C |

Optionally, the IL-15 comprises: IL-15 and mutations, truncations, and various derivatives thereof that can bind to IL-15Ra; the IL-15Ra comprises: IL-15Ra and mutations, truncations, and various derivatives thereof that can bind to IL-15; preferably, the IL-15 includes but is not limited to any combination of the following mutation methods, with the counting method starting to count the first amino acid of the IL-15 sequence as the first position;

| Combinations | **IL-15 mutations** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |
| 21 | D30N/E64Q/N65D |

,
Alternatively, the IL-15/IL-15Ra complex includes but is not limited to any combination of the following mutation methods, with the counting method starting to count the first amino acid of the IL-15 or IL-15Ra sequence as the first position;

| **Combinations** | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C. |

Optionally, the TAA is selected from CD20, CD19, CD30, CD33, CD38, CD40, CD52, slamf7, GD2, CD24, CD47, CD133, CD217, CD239, CD274, CD276, CEA, Epcam, Trop2, TAG72, MUC1, MUC16, mesothelin, folr1, CLDN18.2, EGFR, EGFR VIII, C-MET, HER2, FGFR2, FGFR3, PSMA, PSCA, EphA2, ADAM17, 17-A1, NKG2D ligands, MCSP, LGR5, SSEA3, SLC34A2, BCMA, GPNMB, CCR4, VEGFR-2, CD6, integrin α4, PDGFRα, NeuGcGM3, integrin αVβ3, CD51, CTAA16.88, CD22, ROR1, CSPG4, SS1 or IGFR1.

Optionally, the Fc domain is selected from Human IgG1 Fc, Human IgG2 Fc, Human IgG3 Fc, Human IgG4 Fc or variants thereof, preferably IgG1 Fc, or Human IgG4 Fc or variants thereof; the Fc domain is in the form of a Fc heterodimer; preferably, the Fc heterodimer includes but is not limited to a combination of the following mutations, which are counted according to EU numbering:

| Combinations | FC | Heterodimer mutations (Eu numbering) |
|---|---|---|
| 1 | The first FC chain | T366Y |
| | The second FC chain | Y407T |
| 2 | The first FC chain | T366W |
| | The second FC chain | T366S/L368A/Y407V |
| 3 | The first FC chain | S354C/T366W |
| | The second FC chain | Y349C/T366S/L368A/Y407V |
| 4 | The first FC chain | S364H/F405A |
| | The second FC chain | Y349T/T394F |
| 5 | The first FC chain | T350V/L351Y/F405A/Y407V |
| | The second FC chain | T350V/T366L/K392L/T394W |
| 6 | The first FC chain | K392D/K409D |
| | The second FC chain | E356K/D399K |
| 7 | The first FC chain | D221 E/P228E/L368E |
| | The second FC chain | D221 R/P228R/K409R |
| 8 | The first FC chain | K360E/K409W |
| | The second FC chain | Q347R/D399V/F405T |
| 9 | The first FC chain | K360E/K409W/Y349C |
| | The second FC chain | Q347R/D399V/F405T/S354C |
| 10 | The first FC chain | K370E/K409W |
| | The second FC chain | E357N/D399V/F405T |
| 11 | The first FC chain | F405L |
| | The second FC chain | K409R |
| 12 | The first FC chain | K360D/D399M/Y407A |
| | The second FC chain | E345R/Q347R/T366V/K409V |
| 13 | The first FC chain | Y349S/K370Y/T366M/K409V |
| | The second FC chain | E356G/E357D/S364Q/Y407A |
| 14 | The first FC chain | L351 D/L368E |
| | The second FC chain | L351 K/T366K |
| 15 | The first FC chain | GQPFRPEVHLLPPSREEMTKNQVSLTCLARGFYPK DIAVEWESNGQPENNYKTTPSRQEPSQGTTTFAVT SKLTVDKSRWQQGNVFSCSVMHEALHNHYTQKTI SL |
| | The second FC chain | GQPREPQVYTLPPPSEELALNELVTLTCLVKGFYP SDIAVEWLQGSQELPREKYLTWAPVLDSDGSFFLY |
| | | SILRVAAEDWKKGDTFSCSVMHEALHNHYTQKSLD R |
| 16 | The first FC chain | L368D/K370S |
| | The second FC chain | E357Q/S364K |
| 17 | The first FC chain | S354C/T366W/**K409A** |
| | The second FC chain | Y349C/T366S/L368A/Y407V/**F405K** |
| 18 | The first FC chain | S354C/T366W/**F405K/K360EQ347E** |
| | The second FC chain | Y349C/T366S/L368A/Y407V/**Q347R/T394W** |
| 19 | The first FC chain | T366W/**K409A** |
| | The second FC chain | T366S/**L368G/Y407A/F405K** |
| 20 | The first FC chain | knobs(T366W/**F405K**) |
| | The second FC chain | holes(T366S/**L368G/Y407A/K409A)** |
| 21 | The first FC chain | Q347A/S364K/T366V/K370T/K392Y/F405S/Y407V/K 409W/T411N |
| | The second FC chain | Q347E/Y349A/L351F/S364T/T366V//K370T/T394D/V 397L/D399E/D401Q/F405A/Y407S/K409R/T411R |
| 22 | The first FC chain | K274Q/N276K/Y300F/A339T/Q347A/S364K/T366V/ K370T/N384S/K392Y/V397M/F405S/Y407V/K409W/ T411N/V422I/H435R/Y436F |
| | The second FC chain | Q347E/Y349A/L351F/S364T/T366V//K370T/T394D/V 397L/D399E/D401Q/F405A/Y407S/K409R/T411R. |

Optionally, the Fc domain selectively eliminates immune effector functions, preferably comprises any one of the following mutation modes, which are counted according to EU numbering:

| **IgG** | FC Mutate(EU numbing) |
|---|---|
| **IgG1** | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234A,L235A,P331S |
| | L234A,L235A,N297A |
| | L234A L235A D265A |
| | L235E,D265A,P331S |
| | L235E,N297A,P331S |
| | L235E,N297A |
| | L235A,D265A,P331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

Optionally, the first Fc chain does not bind to protein A, preferably comprises mutations H435R or H435R/Y436F, according to EU numbering; the second Fc chain can bind to protein A.

Optionally, the CD16A binding region comprises a VH domain shown in SEQ ID NO: 97 and a VL domain shown in SEQ ID NO: 96; alternatively, the sequence of IL-15 is shown in SEQ ID NO: 104 or 105; alternatively, the sequence of IL-15Ra may be represented by any one of SEQ ID NOs: 106 to 112; preferably, there is at least one pair of disulfide bond between VH and VL in the CD16A binding region, and/or between IL-15 and IL-15Ra.

Optionally, it is obtained by fusing amino acid fragments shown in any one of the following sets of sequences or amino acid fragments with over 90% identity:
(1) SEQ ID NO: 01, 02, 03, 04;
(2) SEQ ID NO: 01, 05, 03, 06;
(3) SEQ ID NO: 07, 08, 09, 10;
(4) SEQ ID NO: 01, 02, 11, 12;
(5) SEQ ID NO: 01, 02, 13, 14;
(6) SEQ ID NO: 01, 05, 11, 15;
(7) SEQ ID NO: 01, 05, 13, 16;
(8) SEQ ID NO: 38, 39, 40, 41;
(9) SEQ ID NO: 38, 39, 42, 43;
(10) SEQ ID NO: 38, 39, 44, 45;
(11) SEQ ID NO: 38, 39, 46, 47;
(12) SEQ ID NO: 48, 49, 50, 51;
(13) SEQ ID NO: 48, 49, 52, 53;
(14) SEQ ID NO: 48, 49, 54, 55;
(15) SEQ ID NO: 48, 49, 56, 57;
(16) SEQ ID NO: 58, 59, 60, 61;
(17) SEQ ID NO: 58, 59, 62, 63;
(18) SEQ ID NO: 58, 59, 64, 65;
(19) SEQ ID NO: 58, 59, 66, 67;
(20) SEQ ID NO: 68, 69, 70, 71;
(21) SEQ ID NO: 72, 73, 70, 71;
(22) SEQ ID NO: 74, 75, 70, 71;
(23) SEQ ID NO: 76, 77, 70, 71;
(24) SEQ ID NO: 78, 79, 70, 71;
(25) SEQ ID NO: 38, 39, 80, 81;
(26) SEQ ID NO: 38, 39, 82, 83;
(27) SEQ ID NO: 38, 39, 84, 85;
(28) SEQ ID NO: 38, 39, 86, 87;
(29) SEQ ID NO: 38, 39, 88, 89;
(30) SEQ ID NO: 38, 39, 90, 91;
(31) SEQ ID NO: 38, 39, 92, 93;
(32) SEQ ID NO: 38, 39, 94, 95.

Optionally, the antigen also includes infectious disease related antigens, pathogens, and immune function related antigens. When targeting an antigen other than TAA, the TAA binding region of the trifunctional fusion protein that specifically binds to tumor associated antigen (TAA) is replaced with the corresponding antigen binding region.

The present invention also provides a nucleic acid molecule that encodes the antigen targeting, anti-CD16A, and immune effector cell activating trifunctional fusion protein.

The present invention also provides a pharmaceutical composition, which comprises the antigen targeting, anti-CD16A, and immune effector cell activating trifunctional fusion protein, and pharmaceutically acceptable carriers.

The present invention also provides a use of the antigen targeting, anti-CD16A, and immune effector cell activating trifunctional fusion protein in the preparation of drugs for inhibiting or treating cancer, infection, and immunomodulatory diseases.

The cancer includes prostate cancer, lung cancer, colon cancer, rectal cancer, bladder cancer, melanoma, kidney cancer, oral cancer, pharyngeal cancer, pancreatic cancer, uterine cancer, thyroid cancer, skin cancer, head and neck cancer, cervical cancer, ovarian cancer or hematological system cancer.

The present invention further provides a protein complex unit, comprising: the CD16A antibody variable region and the cytokine of the third peptide chain, the CD16A antibody variable region and the cytokine of the fourth peptide chain in the antigen targeting, anti-CD16A, and immune effector cell activating trifunctional fusion protein, and several connecting fragments; the CD16A antibody variable region of the third peptide chain is fused with the cytokine of the third peptide chain through a connecting fragment; the CD16A antibody variable region of the fourth peptide chain is fused with the cytokine of the fourth peptide chain through a connecting fragment; the CD16A antibody variable region of the third peptide chain and the CD16A antibody variable region of the fourth peptide chain are selected from the VH domain or VL domain targeting CD16A, and pair to form a FV fragment; the cytokine of the third peptide chain and the cytokine of the fourth peptide chain are selected from IL15 or IL15Rα, and interact to form an IL15/IL15Rα complex; preferably, there are more than one pair of disulfide bonds between the VH domain and the VL domain, and/or between the IL15 and IL15Rα.

The present invention also provides a nucleic acid molecule that encodes the protein complex unit.

The beneficial effects of the invention:
(1) The trifunctional fusion protein provided by the present invention includes a CD16A binding region that specifically binds to CD16A, a TAA binding region that specifically binds to tumor associated antigen (TAA), an IL-15/IL-15Rα complex formed by the interaction of IL-15 and IL-15Rα, and an Fc domain. It not only targets CD16A and TAA simultaneously, but also fuses the IL-15/IL-15Rα complex and Fc domain, and it has unique components and forms a unique configuration.
(2) The TAA/CD16A/IL15 trifunctional fusion protein designed by the present invention targets NK cells to the tumor by simultaneously binding to tumor associated antigens (TAA) on tumor cells and CD16A on NK cells. NK cells release perforin and granzyme, leading to cell apoptosis and tumor cell death. The cytokine IL15 makes NK cell expansion and maintains functions of NK cells, stimulates immune cell proliferation, and alters the immune microenvironment of tumors.
(3) The antigen targeting, anti-CD16A, and immune effector cell activating trifunctional fusion protein constructed by the present invention has IL-15/IL-15Rα activity. It can target cytokines to the tumor site, specifically expand and activate immune effector cells (NK cells and T cells) at the tumor site, and increase the number of immune cells and the release of killing cytokines, thus more effectively activating the immune response to kill tumor cells.
(4) The CL and CH1 domains in the antibody structure are replaced with IL-15 and IL-15Rα, utilizing the high affinity of IL-15 and IL-15Rα to form the IL-15/IL-15Rα complex, thereby achieving correct pairing of bispecific antibody light chains and solving the problem of bispecific antibody light chain mismatch. In addition, one or more pairs of disulfide bonds can be added between VH and VL, as well as between IL-15 and IL-15Rα by amino acid sequence mutations, to further enhance the binding activity between light and heavy chains, and by combining with Fc heterodimer technology, the problem of heavy chain mismatch in bispecific antibodies can be solved, thereby effectively overcoming challenges such as light and heavy chain mismatch, high byproducts, and poor stability in the preparation of bispecific antibodies, and obtaining correctly paired multifunctional fusion proteins. At the same time, the development cycle of multifunctional fusion proteins can be shortened and the production costs can be saved.
(5) The functional effects of the Fc domain can be retained, or the Fc domain in the form of eliminating immune effects can be adopted. Using the Fc domain in the form of eliminating immune effects prevents Fc from binding to FcR y III (CD16A). If the fusion protein of the present invention retains the effector function of the Fc domain, the corresponding activation effect of immune cells can also be observed.

### DESCRIPTION OF THE DRAWINGS

Figures 1 to 19 show the structural diagrams of the trifunctional fusion proteins in different embodiments of the present invention, respectively; wherein, Figures 1 to 9 show the trifunctional fusion proteins in the form of the ratio of the TAA binding region and CD 16A binding region is 1:1; Figures 10 to 19 show the trifunctional fusion proteins in the form of the ratio of the TAA binding region and CD 16A binding region is 2:1.
Figure 20 Figure 21 shows the results of FACS detection of trifunctional proteins binding to HL60 cells.
Figure 22, Figure 23 and Figure 24 show the results of FACS detection of CD38/CD16A/IL-15 trifunctional fusion proteins binding to Daudi cells.
Figure 25 shows the results of ELISA detection of trifunctional molecules binding to CS1 protein.
Figures 26 to 32 show the results of CCK8 detection of TAA/CD16A/IL-15 trifunctional fusion protein on Mo7e cell proliferation experiment.
Figures 33 to 38 show the results of ADCC effect mediated by the CD33/CD16A/IL-15 trifunctional fusion protein, wherein the effector-target ratio is 10:1 in Figure 33 and Figure 34, the effector-target ratio is 20:1 in Figure 35, and the effector-target ratio is 10:1 in Figure 36, Figure 37 and Figure 38.
Figures 39 to 42 show the results of ADCP mediated by the CD38/CD16A/IL-15 trifunctional fusion protein, wherein the effector-target ratio is 10:1 in Figure 39, the effector-target ratio is 5:1 in Figure 40, and the effector-target ratio is 10:1 in Figures 41 and 42.
Figure 43 shows the results of ADCP mediated by the CD33/CD16A/IL-15 trifunctional fusion protein.

### Detailed Description

The technical solution of the present invention is further described below in conjunction with drawings and examples. In order to make this disclosure easier to understand, certain technical and scientific terms are specifically defined below. Unless clearly defined elsewhere herein, all other technical and scientific terms used herein have the meanings commonly understood by those skilled in the art.

### Terms

The term "fusion" refers to the direct connection of components by peptide bonds or through connecting fragments, or non-covalent fusion through intermolecular interactions, or covalent fusion through one or more disulfide bonds or chemical cross-linking. In a single peptide chain, fusion refers to the direct connection by peptide bonds or through connecting fragments. A "multifunctional fusion protein" refers to a protein that contains two or more antigen-binding domains, capable of binding to two or more different epitopes (such as two, three, or more different epitopes). A multifunctional fusion protein may further contain cytokines (such as IL-15, IL-15Ra), etc. "Fusion position" refers to the position of a functional region or domain in a peptide chain, indicating the order of connection of various functional segments on the peptide chain.

The term "polypeptide" refers to any length of amino acid chain, including proteins and fragments thereof. Polypeptides are disclosed as amino acid sequences in the present invention. Those sequences are written from left to right in the direction from the amino end to the carboxyl end. According to the standard naming convention, amino acid residue sequences are named with three or single letter codes, as follows: alanine (Ala, A), arginine (Arg, R), asparagine (Asn, N), aspartate (Asp, D), cysteine (Cys, C), glutamine (Gln, Q), glutamic acid (Glu, E), glycine (Gly, G), histidine (His, H), isoleucine (I1e, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

The term "peptide chain" refers to a molecule that is formed by linearly linking amino acids by peptide bonds.

The term "variant" or "mutant" refers to a polypeptide or polynucleotide that differs from the contained amino acids or nucleotides but maintains its basic characteristics. The differences between variants or between a variant and its maternal antibody are usually limited, and the amino acid sequences are generally very similar. In this specification, the antibody or antibody fragment before mutation is referred to as maternal antibody, and the mutated antibody or antibody fragment is referred to as variant. The variant still has antigen binding activity.

The term "antibody (Ab)" refers to an immunoglobulin molecule (Ig) that contains at least one antigen binding site and can specifically bind to an antigen.

The term "antigen" refers to a substance in the body that can induce an immune response and specifically bind to antibodies. The binding of antibodies to antigens relies on the interaction formed between the two, including hydrogen bonds, van der Waals' force, ionic bonds, and hydrophobic bonds. The region where the antigen surface binds to the antibody is called the "antigenic determinant cluster" or "epitope". Generally speaking, each antigen has multiple determinant clusters.

The term "antibody" referred to in the present invention is understood in its widest sense and includes monoclonal antibodies (including full-length monoclonal antibodies), polyclonal antibodies, antibody fragments, and multispecific antibodies containing at least two different antigen-binding domains (e.g., bispecific antibodies). Antibodies also include mouse-derived antibodies, humanized antibodies, chimeric antibodies, human antibodies, and antibodies derived from other origins. The antibodies of the present invention can originate from any animal, including but not limited to immunoglobulin molecules of humans, non-human primates, mice, rats, cows, horses, chickens, camels, alpacas, etc. Antibodies can contain additional changes, such as non-natural amino acids, Fc effector functional mutations, and glycosylation site mutations. Antibodies also include post translational modified antibodies, fusion proteins containing antigenic determinant clusters of antibodies, and any other modified immunoglobulin molecules containing antigenic recognition sites, as long as these antibodies exhibit the desired biological activity. In other words, antibodies include immunoglobulin molecules and immune active fragments thereof, i.e., a molecule containing at least one antigen-binding domain.

The basic structure of an antibody is a Y-shaped monomer connected by two identical heavy chains (H) and two identical light chains (L) via disulfide bonds. Each chain is composed of 2-5 structural domains (also known as functional regions) containing approximately 110 amino acids, with similar sequences but different functions. The amino acid sequences near the N-terminus of the light and heavy chains in an antibody molecule undergo significant changes, forming a structural domain called the variable region (V region); and the region near the C-terminus where the amino acid sequences are relatively constant is called the constant region (C region).

The V regions of heavy and light chains are called VH and VL, respectively. VH and VL each have three regions of highly variable amino acid composition and arrangement, known as the hypervariable region (HVR). This region forms a spatial conformation complementary to the antigen epitope, also known as the complementarity determining region (CDR). The three CDRs of VH are represented by VHCDR1, VHCDR2, and VHCDR3, while the three CDRs of VL are represented by VLCDR1, VLCDR2, and VLCDR3, respectively. VH and VL have a total of 6 CDRs that together form the antigen-binding site. The diversity of amino acids in the CDR region is the molecular basis for the specific binding of antibodies to a large number of different antigens. The composition and arrangement order of amino acids outside of CDR in the V region have relatively little change, and are called the framework region (FR). VH and VL each have four skeleton regions (or framework regions), represented by FR1, FR2, FR3, and FR4, respectively. VH and VL each consist of three CDRs and four FRs, arranged from the amino end to the carboxyl end in the following order: FR1, CDR1, FR2, CDR2, FR3, CDR3, FR4.

According to the amino acid sequence of the antibody heavy chain constant region, human immunoglobulins can be divided into 5 categories: IgM, IgG, IgA, IgD, and IgE. It can also be further divided into different subtypes (isotypes), such as human IgG can be divided into IgG1, IgG2, IgG3, IgG4; IgA can be divided into IgA1 and IgA2. No subtypes have been found for IgM, IgD, and IgE. According to the amino acid sequence of light chain, light chains can be classified as κ chain and λ chain. The antibodies of the present invention can be of any type (such as IgM, IgG, IgA, IgD, IgE) or subtype (such as IgG1, IgG2, IgG3, IgG4, IgA1, IgA2).

The constant regions of heavy and light chains are called CH and CL, respectively. The heavy chain constant regions of IgG, IgA, and IgD have three structural domains: CH1, CH2, and CH3, while the heavy chain constant regions of IgM and IgE have four structural domains: CH1, CH2, CH3, and CH4.

The hinge region is located between CH1 and CH2 and is rich in proline, making it easy to stretch and bend. It can change the distance between the two arms in a Y-shape, which is beneficial for both arms to simultaneously bind to antigenic epitopes.

The term "Fab fragment" is a fragment of antigen binding (Fab), which refers to an antibody fragment consisting of VL, VH, CL and CH1 domains, and it binds to a single antigenic epitope (monovalent). Those skilled in this field know that under certain conditions, certain parts of the antibody molecular chain are easily hydrolyzed into various fragments by proteolytic enzymes. Papain hydrolyzes an antibody molecule into two identical antigen binding fragments (Fab) and one crystallizable fragment (Fc) from the near N-terminus of the hinge region.

The term "Fv fragment" refers to a monovalent small molecule composed of the V region (VH+VL) of the L and H chains, which is the smallest functional fragment that binds to an antigen.

The terms "Fc", "Fc segment", "Fc fragment", and "Fc domain" refer to a fragment crystallizable that has no antigen binding activity and is the site where antibodies interact with effector molecules or cell surface Fc receptors (FcR). Fc fragments bind to cells with corresponding Fc receptors on the surface, producing different biological effects. In ADCC effect (antibody dependent cell-mediated cytotoxicity), the Fab segment of the antibody binds to the antigenic epitopes of virus infected cells or tumor cells, and its Fc segment binds to the FcR on the surface of killer cells (NK cells, macrophages, etc.), mediating direct killing of target cells by killer cells. The Fc fragment contains constant region peptides of the antibody, except for the heavy chain constant region CH1, namely the two constant region domains CH2 and CH3 of the carboxyl end of the heavy chain constant region of human immunoglobulin IgA, IgD, and IgG, as well as the three constant region domains CH2, CH3, and CH4 of the carboxyl end of the heavy chain constant region of human immunoglobulin IgE and IgM. The Fc fragment is often selected from Human IgG1 Fc, Human IgG2 Fc, Human IgG3 Fc, Human IgG4 Fc or their variants, preferably from IgG1 Fc, or Human IgG4 Fc or variants thereof.

Fc can be composed of two chains. Herein, the two chains of Fc fragments are described as the first Fc chain and the second Fc chain. The first Fc chain and the second Fc chain can be mutated separately, and it is not particularly limited in the present invention. Fc fragment can also refer to a single polypeptide chain in the Fc domain. The Fc segment of antibodies can selectively eliminate immune effector functions, including but not limited to combinations of the following mutations (according to EU numbering)

| **IgG** | FC Mutate(EU numbing) |
|---|---|
| **IgG1** | L234A,L235A |
| | L234A,L235A,P329G |
| | L234F,L235E,P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234A,L235A,P331S |
| | L234A,L235A,N297A |
| | L234A,L235A,D265A |
| | L235E,D265A,P331S |
| | L235E,N297A,P331S |
| | L235E,N297A |
| | L235A,D265A,P331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

The Fc variants designed through mutation can produce spatial filling effects, electrostatic steering, hydrogen bonding, hydrophobic interactions, and so on. The interaction between Fc variants is conducive to form a stable heterodimer. A preferred mutation design is a type of "Knob-in-hole" mutation design.

A "connecting fragment" refer to one or more amino acid residues inserted into the immunoglobulin domain and providing sufficient mobility, ensuring the correct folding and peptide stability of the protein. A "connecting fragment" is preferably as (GGGGS) n, wherein n may be 0, 1, 2, 3, 4, 5, or more. If the connecting fragment sequence is too short, it may affect the folding of the advanced structure of the two proteins, thereby interfering with each other. If the connecting peptide sequence is too long, it may involve immunogenicity issues, because the connecting peptide sequence itself is a new antigen.

Tumor-associated antigen (TAA) refers to an antigen molecule presents on tumor cells or normal cells, including embryonic proteins, glycoprotein antigens, squamous cell antigens, etc. It is commonly used in the clinical diagnosis of tumors. Tumor-associated antigens are not specific to tumor cells, but can be synthesized by normal cells in trace amounts, and are highly expressed when tumor cells proliferate, so they are called "associated antigens". Tumor-associated antigens may be, such as CD20, CD19, CD30, CD33, CD38, CD40, CD52, slamf7, GD2, CD24, CD47, CD133, CD239, CD276, PD-1, CEA, Epcam, Trop2, TAG72, MUC1, MUC16, mesothelin, folr1, CLDN18.2, PDL1, EGFR, EGFR VIII, C-MET, HER2, FGFR2, FGFR3, PSMA, PSCA, EphA2, ADAM17, 17-A1, NKG2D ligands, MCSP, LGR5, SSEA3, SLC34A2, BCMA, GPNMB, or Glypican-3.

The term "vector" refers to a polynucleotide molecule that can transport another polynucleotide connected to it. One type of vector is a "plasmid", which is a circular double stranded DNA loop, in which additional DNA segments can be connected. Another type of vector is a viral vector, in which additional DNA segments can be connected to the viral genome. Some vectors can autonomously replicate in the host cells where they are introduced (for example, bacterial vectors with bacterial origin of replication and additive mammalian vectors). Other vectors (such as non-additive mammalian vectors) can be integrated into the host cell's genome after being introduced into the host cell, thereby replicating together with the host genome. In addition, certain vectors can guide the expression of genes that can are operably connected to them. Usually, useful expression vectors in recombinant DNA technology are in the form of plasmids. The present invention can provide a vector containing a nucleic acid fragment capable of encoding a trifunctional fusion protein.

The terms "IL-15" and "IL-15Ra" may refer to their mutants or fragments. The "IL-15" described in the present invention may be any IL-15 or mutants that can combine with IL-15Rα, such as human IL-15 or non-human mammalian or non-mammalian IL-15. Exemplary non-human mammals are such as pigs, rabbits, monkeys, chimpanzees, mice, etc., and non-human mammals such as chickens; preferably human IL-15. The term "mutant that can combine with IL-15Rα" refers to a mutant molecule that increases or decreases the affinity between IL-15 and its receptors, or increases or decreases the activity of IL-15 in stimulating T or NK cells, which is obtained through one or more amino acid substitution, addition or deletion mutations.

The "IL-15Rα" described in the present invention may be IL-15Rα of any species or mutants that can combine with IL-15Rα, such as human IL-15Rα or non-human mammalian IL-15Rα or non-mammalian IL-15Rα. Exemplary non-human mammals are such as pigs, rabbits, monkeys, chimpanzees, mice, etc., and non-human mammals such as chickens. Preferably human IL-15Rα, more preferably human IL-15Rα extracellular domain fragment, abbreviated as IL-15Rα ECD (see UniProtKB database, accession number Q13261, 31-205aa). The term "mutant can be combined with IL-15Rα" refers to a functional mutant generated through one or more amino acids deletion, insertion, or substitution on IL-15Rα, with the ability to bind to its ligand molecules such as IL-15, preferably human IL-15Rα molecule, more preferably the shortened form of human IL-15Rα extracellular domain fragment, which is a molecule obtained through one or more amino acid deletion mutations starting from the C-terminus of the extracellular domain fragment, with the activity of IL-15 reporter α, preferably the deletion mutation form retaining 65-178 amino acids, such as IL-15Rα. The IL-15 protein itself is not as stable as when combined with IL-15Rα protein. As known in this field, IL-15Rα protein contains a "sushi domain", which is the shortest region of the receptor that retains IL-15 binding activity, or a domain in the extracellular domain that can maintain over 90% binding activity.

CS1 (also known as CD319, SLAMF7) is signaling lymphocytic activation molecule family member 7, which is a surface receptor protein that regulates NK cell immune function. CS1 is highly expressed in multiple myeloma (MM) cell lines, but has not been found in healthy tissues, primary tumor tissues, or hematological and non-hematological cancer cell lines, making it an attractive target for treating said disease.

CD33 is a 67kD single channel transmembrane glycoprotein and a member of the sialic acid binding immunoglobulin-like lectins (Siglecs) family. Although its exact biological function is not yet clear, in normal individuals, it is mainly considered as a myeloid differentiation antigen, with lower expression in myeloid progenitor cells, neutrophils, and macrophages, and high expression in circulating monocytes and dendritic cells. Importantly, CD33 has been detected in the metrocytes and leukemia stem cells of 85% to 90% of patients with acute myeloid leukemia (AML). Interestingly, the expression of CD33 is limited to hematopoietic cells (Paul, Taylor, Stansbury, and McVicar, 2000; Ulyanova, Blasili, Woodford Thomas, and Thomas, 1999), but it is not present in normal hematopoietic stem cells (Andrews, Torok Storb, and Bernstein, 1983; Griffin, Linch, Sabbath, Larcom, and Schlossman, 1984; Jilani et al., 2002). These findings suggest that CD33 is a suitable target for antibody-based therapies in AML.

CD38 antigen was discovered in 1980 and is a type II transmembrane glycoprotein with a size of 46 kDa. The ligand of CD38 is CD31, also known as PECAM-1, which is a member of the Ig gene superfamily. CD31 is not only expressed on endothelial cells, but also in lymphoid cells (follicular membrane B cells and plasma cells), lungs (alveolar ducts, alveoli, and lymphatic vessels), and kidneys (glomerular cells). The interaction between CD38 and ligand CD31 plays an important role in regulating cell migration, receptor mediated adhesion, and signal transduction. Moreover, CD38 protein is also a bifunctional extracellular enzyme that combines the activities of cyclases and hydrolases, participating in nucleotide metabolism. CD38 uses NAD+ as a substrate to form cyclic ADP ribose (cADPR) and ADPR. These nucleotide metabolites are effective second messengers that regulate the mobilization of calcium ions in the cytoplasm, activate signaling pathways that control various biological processes, such as lymphocyte proliferation and insulin secretion by pancreatic B cells. Further studies have shown that the production of CD38 dependent adenosine also plays a crucial role in immune suppression mediated by natural killer (NK) cells. Usually, CD38 is expressed at low levels in normal lymphoid and myeloid cells, as well as non hematopoietic tissues, while it is widely expressed in cells of hematopoietic origin (including committed stem cells). CD38 is expressed in B cells, while the expression level of CD38 is particularly high in plasma cells (also known as effector B cells). The expression of CD38 is associated with many diseases, including AIDS, autoimmune diseases (such as systemic lupus erythematosus), type 2 diabetes, osteoporosis and cancer. Researches have found that CD38 is highly expressed in a large number of malignant hematological cancers, especially in cancers such as multiple myeloma, making it a target for the development of therapeutic antibody drugs for multiple myeloma.

"Effector function" refers to biochemical events generated by the interaction between the Fc region of antibodies and Fc receptors or ligands. The effector functions include but are not limited to ADCC, ADCP, and CDC. "ADCC" or "antibody dependent cell-mediated cytotoxicity" refers to cell-mediated reactions in which the non-specific cytotoxic cells expressing FcyR recognize binding antibodies on target cells and subsequently cause target cell lysis. ADCC is associated with the binding with FcyRIIIa; and the increased binding with FcyRIIIa leads to an increase in ADCC activity. As discussed herein, in many examples of the present invention, the ADCC activity is completely eliminated. "ADCP" or antibody dependent cell-mediated phagocytosis refers to a cell-mediated response in which non-specific cytotoxic cells expressing FcyR recognize binding antibodies on target cells and subsequently induce phagocytosis of target cells.

In terms of protein sequences, "percentage (%) of amino acid sequence identity" is defined as the percentage of amino acid residues in a candidate sequence that are identical to those in a specific (parent) sequence, when aligning sequences and introducing gaps as necessary to achieve maximum percentage sequence identity, without considering any conservative substitutions as parts of sequence identity. In some embodiments, two or more amino acid sequences are at least 80%, 85%, or 90% identical. In some embodiments, two or more amino acid sequences are at least 95%, 97%, 98%, 99%, or even 100% identical.

The terms "first" and "second" are only used for descriptive purposes and do not indicate or imply their level of importance.

The antigen targeting, anti-CD16A, and immune effector cell activating trifunctional fusion protein of the present invention comprises: CD16A binding region that specifically binds to CD16A, TAA binding region that specifically binds to tumor associated antigen (TAA), IL-15/IL-15Rα complex formed by the interaction of IL-15 and IL-15Rα, and Fc domain. The TAA/CD16A/IL-15 trifunctional fusion protein of the present invention exerts therapeutic effects mainly by simultaneously binding to tumor associated antigens (TAA) expressed on tumor cells and CD 16A on NK cells, cross-linking these two types of cells to form immune synapses, promoting NK cells to kill tumor cells, while IL-15 expands and maintains NK cell function.

In some embodiments, the TAA binding region and CD16A binding region of the fusion protein of the present invention is in the form of 1:1; in other embodiments, is in the form of 2:1.

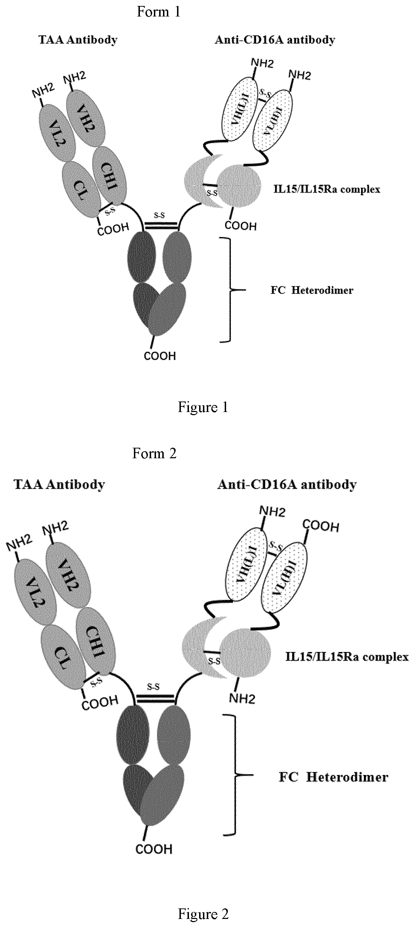

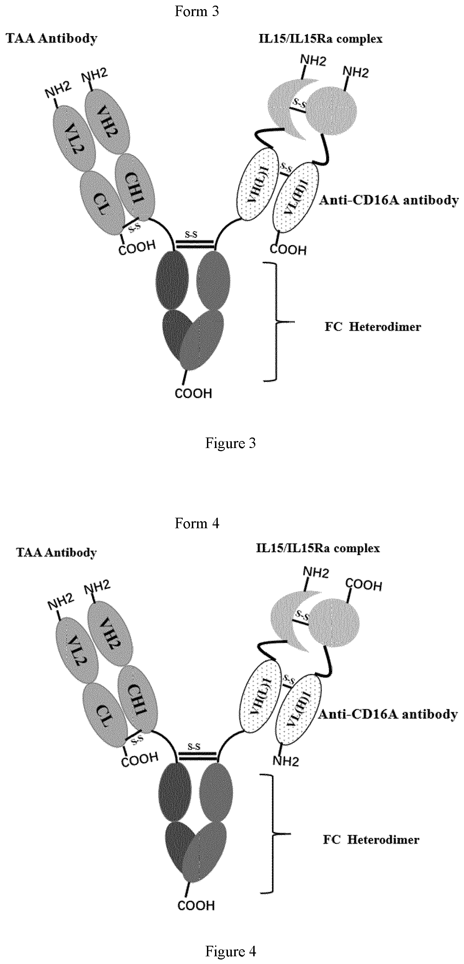

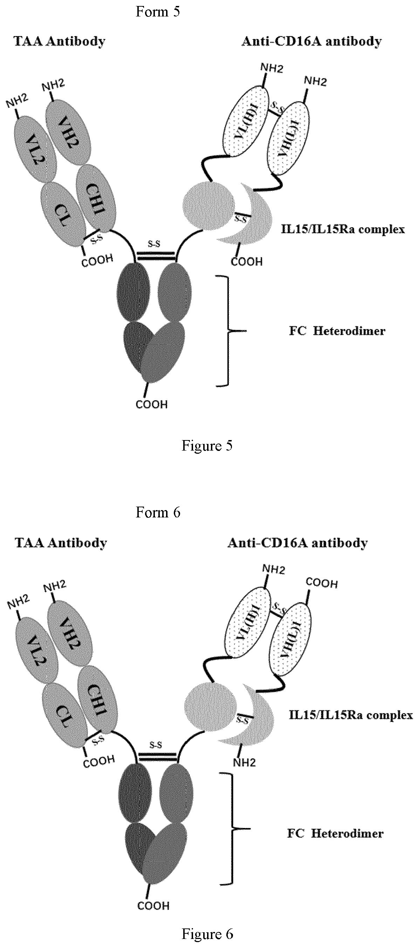

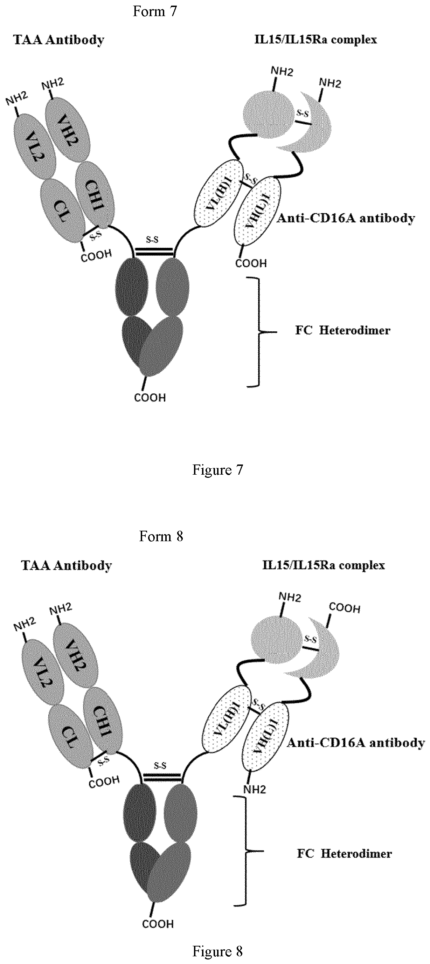

The structures of the trifunctional fusion protein in the form of 1:1 can be found in Figures 1-9. The fusion protein is formed by the fusion of four peptide chains, with the first and second peptide chains forming the first monomer (left part of the figure), and the third and fourth peptide chains forming the second monomer (right part of the figure). The first peptide chain comprises a light chain formed by the fusion of the VL domain and the CL domain, while the second peptide chain comprises a heavy chain formed by the fusion of the VH domain, CH1 domain, and the first Fc chain. The VL and CL domains of the first peptide chain, as well as the VH and CH1 domains of the second peptide chain, are paired to form a Fab fragment. The third peptide chain comprises: the antibody variable region, the cytokine, and the second Fc chain. The fourth peptide chain comprises: the antibody variable region and the cytokine. The antibody variable region of the third peptide chain pairs with the antibody variable region of the fourth peptide chain to form a Fv fragment, and the cytokine of the third peptide chain interacts with that of the fourth peptide chain to form the IL-15/IL-15Rα complex.

In the fusion proteins shown in Figures 1 to 8, the Fab fragment in the first monomer targets TAA, achieving the function of TAA Antibody as shown in the figures. In the second monomer, the antibody variable region that pairs to form the Fv fragment is selected from the VH and VL domains. For example, the antibody variable region in the third peptide chain selects the VH domain targeting CD16A, the antibody variable region in the fourth peptide chain selects the VL domain targeting CD16A, and the VH and VL pair to form the Fv fragment that targets CD16A, achieving the function of Anti CD16A Antibody as shown in the figures. In other embodiments, the antibody variable region in the third peptide chain selects the VL domain targeting CD16A, while the antibody variable region in the fourth peptide chain selects the VH domain targeting CD16A. The VH (L) in the figures represents the selection of VH or VL. The numbers in "VH (L) 1", "VL (H) 1", "VH2", and "VL2" in the figures are only used for descriptive purposes and represent the variable regions of antibodies targeting different antigens. VH (L) 1 represents that VH1 or VL1 can be selected. If VH1 is selected as the variable region of the antibody in the third peptide chain, VL1 should be selected as the variable region of the antibody in the fourth peptide chain, so as to pair and form a Fv fragment. The cytokines in the third and fourth peptide chains are selected from IL-15 and IL-15Rα, to form the IL-15/IL-15Rα complex. In some embodiments, IL-15 is selected as the cytokine in the third peptide chain, and IL-15Rα is selected as the cytokine in the fourth peptide chain. In some embodiments, on the contrary, the third peptide chain selects IL-15Rα, and the fourth peptide chain selects IL-15.

In the third and fourth peptide chains, the fusion order between the antibody variable region (VH or VL) and the cytokine is not limited. For example, in the fusion protein shown in Figure 1, from the N-terminus to the C-terminus of the peptide chain, the fusion order of the third peptide chain is: antibody variable region~cytokine~the second Fc chain, and the fusion order of the fourth peptide chain is: antibody variable region-cytokine, wherein "∼" represents the connecting fragment. The fusion protein shown in Figure 2 differs from Figure 1 in that the fusion sequence of the fourth peptide chain from the N-terminus to the C-terminus of the peptide chain is: cytokine-antibody variable region. The spherical and crescent shapes in Figures 1 to 8 represent different cytokines. Assuming that the spherical shape represents IL-15, the crescent shape represents IL-15Ra. The possible combination forms of the third and fourth peptide chains of the fusion proteins shown in Figures 1 to 8, from the N-terminus to the C-terminus of the peptide chain, are listed below:

**Table 1 Combination forms of the third and fourth peptide chains of fusion proteins**

| | | |
|---|---|---|
| Figure 1 | The third | VH1~IL-15Ra~the second Fc chain |
| | The fourth | VL1~IL-15 |
| Figure 1 | The third | VL1~IL-15Ra~the second Fc chain |
| | The fourth | VH1-IL-15 |
| Figure 2 | The third | VH1~IL-15Ra~the second Fc chain |
| | The fourth | IL-15-VL1 |
| Figure 2 | The third | VL1~IL-15Ra~the second Fc chain |
| | The fourth | IL-15-VH1 |
| Figure 3 | The third | IL-15Ra~VH1~the second Fc chain |
| | The fourth | IL-15-VL1 |
| Figure 3 | The third | IL-15Ra~VL1∼the second Fc chain |
| | The fourth | IL-15-VH1 |
| Figure 4 | The third | IL-15Ra~VH1~the second Fc chain |
| | The fourth | VL1-IL-15 |
| Figure 4 | The third | IL-15Ra~VL1~the second Fc chain |
| | The fourth | VH1-IL-15 |
| Figure 5 | The third | VH1~IL-15~the second Fc chain |
| | The fourth | VL1~IL-15Ra |
| Figure 5 | The third | VL1~IL-15~the second Fc chain |
| | The fourth | VH1~IL-15Ra |
| Figure 6 | The third | VH1~IL-15~the second Fc chain |
| | The fourth | IL-15Ra~VL1 |
| Figure 6 | The third | VL1~IL-15~the second Fc chain |
| | The fourth | IL-15Ra~VH1 |
| Figure 7 | The third | IL-15~VH1~the second Fc chain |
| | The fourth | IL-15Ra~VL1 |
| Figure 7 | The third | IL-15~VL1~the second Fc chain |
| | The fourth | IL-15Ra~VH1 |
| Figure 8 | The third | IL-15~VH1~the second Fc chain |
| | The fourth | VL1~IL-15Ra |
| Figure 8 | The third | IL-15~VL1~the second Fc chain |
| | The fourth | VH1~IL-15Ra |

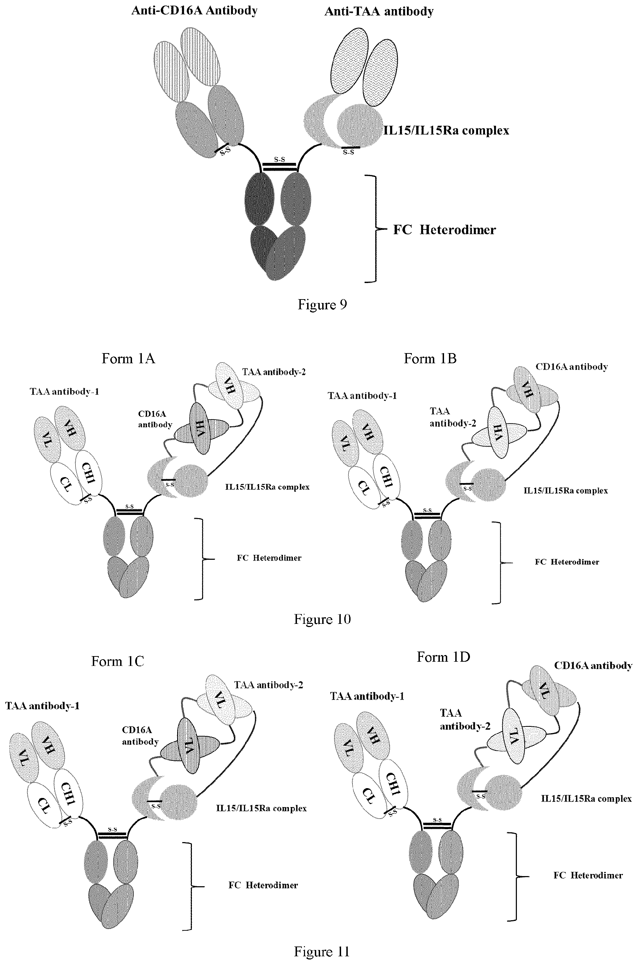

The fusion protein shown in Figure 9 targets CD16A with the Fab fragment in its first monomer (left part of the figure), achieving the function of Anti-CD16A Antibody as shown in the figure. Fv fragment targets TAA to achieve the function of Anti-TAA Antibody as shown in the figure. Similar to the fusion proteins shown in Figures 1 to 8, the fusion protein shown in Figure 9 may also have multiple possible combination forms of the third and fourth peptide chains listed in Table 1.

The structures the trifunctional fusion proteins in the form of the ratio of the TAA binding region and CD16A binding region is 2:1 can be found in Figures 10-19. The fusion protein is formed by the fusion of four peptide chains, with the first and second peptide chains forming the first monomer (left part in the figure), and the third and fourth peptide chains forming the second monomer (right part in the figure). As shown in Figures 10 to 13, in some embodiments, the first monomer comprises a TAA binding region (TAA antibody) and a Fc chain, while the second monomer comprises a CD16A binding region (CD16A antibody), a TAA binding region (TAA antibody), IL-15/IL-15Rα complex (IL15/IL15Ra complex) and a Fc chain, and the two FC chains polymerize to form a Fc domain (Fc heterodimer).

More specifically, the two TAA binding regions are the first TAA binding region and the second TAA binding region, respectively. As shown in Figure 10, in some embodiments, the first peptide chain of the trifunctional fusion protein in the form of 2:1 comprises: a TAA binding antibody variable region (such as a VL domain against TAA) and an antibody constant region (such as the CL domain); the second peptide chain comprises: a TAA binding antibody variable region (such as a VH domain against TAA) and an antibody constant region (such as the CH1 domain and a first Fc chain); the third peptide chain comprises: a TAA binding antibody variable region (such as a VH domain against TAA), a CD16A binding antibody variable region (such as a VL domain against CD16A), a cytokine (such as IL-15), and a second Fc chain; the fourth peptide chain comprises: a CD16A binding antibody variable region (such as a domain against CD16A), a TAA binding antibody variable region (such as a VL domain against TAA), and a cytokine (such as IL-15Ra). The VL and CL of the first peptide chain, as well as the VH and CH1 pairing of the second peptide chain pair to form the first TAA binding region, which is a Fab fragment targeting TAA. The TAA binding antibody variable region of the third peptide chain and the TAA binding antibody variable region of the fourth peptide chain pair form the second TAA binding region, which is a Fv fragment targeting TAA; the CD16A binding antibody variable region of the third peptide chain and the CD16A binding antibody variable region of the fourth peptide chain pair to form the CD16A binding region, which is a Fv fragment targeting CD16A (the second TAA binding region and CD16A binding region form an antibody fragment in the form of diabody); the cytokine of the third peptide chain and the cytokine of the fourth peptide chain interact to form an IL-15/IL-15Rα complex.

The trifunctional fusion proteins (forms 1A to 1D) shown in Figures 10 and 11 target the same target and have the same domain, with the difference being the fusion order of the domains in the third and fourth peptide chains. For example, in Figure 10, from the N-terminus to the C-terminus, the fusion order of the third peptide chain domain of the triple functional fusion protein in form 1A is: VH (anti TAA)~VL (anti CD16A)~IL-15Ra~the second Fc chain; from the N-terminus to the C-terminus, the fusion order of the fourth peptide chain domain is: VH (anti CD16A)~VL (anti TAA)~IL-15. While, from the N-terminus to the C-terminus, the fusion order of the third peptide chain domain of the triple functional fusion protein in form 1B is: VH (anti CD16A)~VL (anti TAA)~IL-15Ra~the second Fc chain; from the N-terminus to the C-terminus, the fusion order of the fourth peptide chain domain is: VH (anti TAA)~VL (anti CD16A)~IL-15.

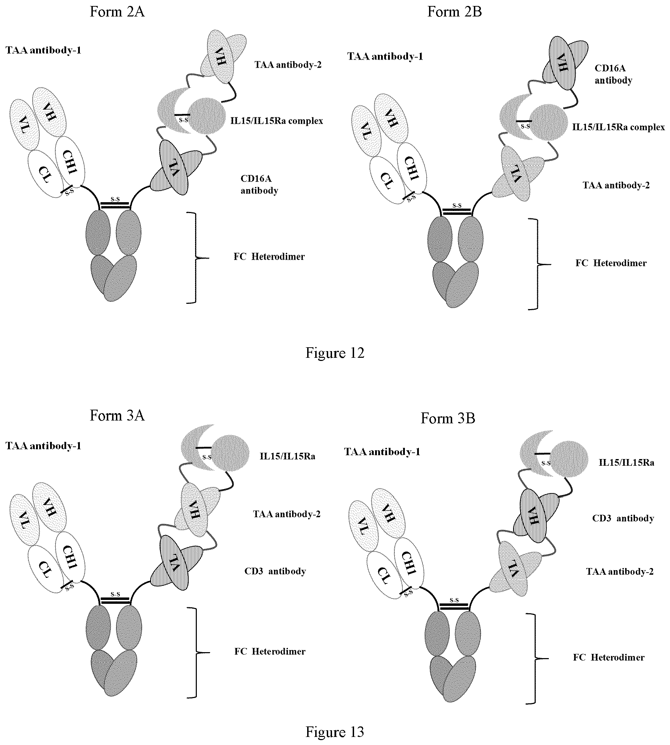

In Figure 12, the trifunctional fusion proteins shown in Forms 2A and 2B are fused with antibody variable regions on both sides of the cytokines. In Figure 13, the trifunctional fusion proteins shown in Forms 3A and 3B are fused with antibody variable regions on the end side of the peptide chains.

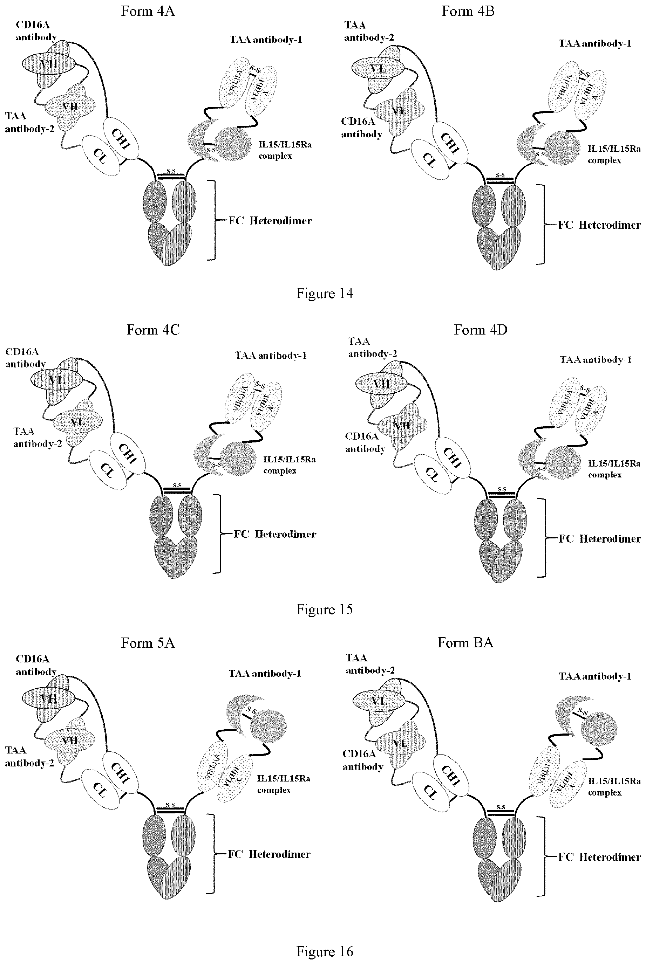

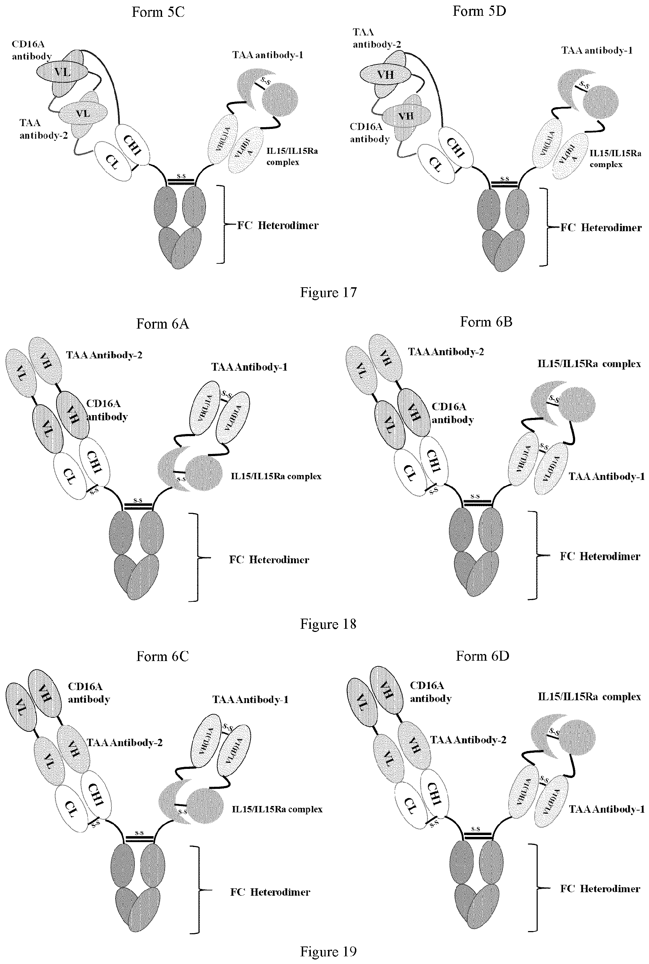

As shown in Figures 14 to 19, in some embodiments, the first monomer comprises a CD16A binding region, a TAA binding region and a Fc chain, the second monomer comprises a TAA binding region, IL-15/IL-15Rα complex and a Fc chain, and the two FC chains polymerize to form a Fc domain. In Figures 14 and 15, the fusion order of the antibody variable regions of the first and second peptide chains of the trifunctional fusion proteins shown in Forms 4A to 4D is different, and cytokines are fused between the antibody variable region and the FC chain. In Figure 16 In Figure 17, the trifunctional fusion proteins shown in Forms 5A and D are fused with cytokines on the end side. In Figure 18 and Figure 19, the trifunctional fusion proteins shown in Forms 6A to 6D, similar to the DVD-Ig bispecific antibody, the VL and VH domains of another antibody are connected to the N-terminus of the light and heavy chains of the antibody in their first monomer, respectively. For example, the fusion order of the first peptide chain domains of the trifunctional fusion protein shown in Form 6A is: VL (anti TAA)~VL (anti CD16A)~CL; the fusion order of its second peptide chain is; VH (anti TAA)~VH (anti CD16A)~CH1~Fc. Its cytokines can be fused between the antibody variable region and the second Fc chain, as well as at the end side.

The present invention does not limit the fusion order of each domain in the fusion protein. Except for the embodiments listed above, those skilled in the art may make other modifications.

The composite unit with an innovative structural protein provided by the present invention can be seen in the upper part of the second monomer of the fusion protein shown in Figure 1, namely the region where the Fv fragment of anti-CD16A fuses with the IL-15/IL-15Rα complex. The composite unit includes two antibody variable regions (VH and VL against CD16A), two cytokines (IL-15, IL-15Ra), and several connecting fragments. One antibody variable region fuses with one cytokine through a connecting fragment. Two antibody variable regions pair to form the Fv fragment, and two cytokines form the IL-15/IL-15R complex through interaction. It is preferred to have more than one pair of disulfide bonds between the VH and VL domains against CD16A, and/or between IL15 and IL15Rα, in order to form a more stable structure. Disulfide bonds can be set between VH and VL, and no disulfide bond can be set between IL15 and IL15Rα. Disulfide bonds can be set between IL15 and IL15Rα, and no disulfide bond can be set between VH and VL. Alternatively, disulfide bonds can be set between VH and VL, as well as between IL15 and IL15Rα. By utilizing the composite units provided by the present invention, those skilled in the art can construct other target proteins, not limited to the forms of fusion proteins illustrated in the examples of the present invention.

There is an extremely high affinity between cytokines IL-15 and IL-15Ra (about 30-100pM KD). In the present invention, IL-15 and IL-15Ra can respectively replace the CL and CH1 domains in one of the antibody structures to solve the problem of light chain mismatch in bispecific antibodies, and heavy chain mismatch can be solved through FC heterodimer form. In some embodiments, the FC end attached to Fab will undergo mutations, making it unable to bind to Protein A. Purification involves a two-step purification process that combines Protein A affinity chromatography at the FC end and CH1 domain CH1-XL affinity chromatography to produce a bispecific targeted antibody.

### Example 1: Molecular Cloning, Transient Expression, and Protein Purification to Obtain TAA/CD16A/IL-15 Trifunctional Fusion Protein

Molecular cloning: In this example, multiple fusion proteins were constructed according to the Table 2 and Table 3, and TAA was selected from CD33, CD38, and CS1, respectively. The protein expression and sequence numbers are shown in the table below. The trifunctional fusion proteins constructed in Table 2 have a 1:1 form of TAA binding region and CD16A binding region; Table 3 shows the construction of the trifunctional fusion proteins. The fusion proteins indicated by the double underlined protein numbers are in a 1:1 form, while the others are in a 2:1 form. Tables 2 and 3 also provide structural descriptions. Taking QP743745 as an example, according to the structural description, it includes four peptide chains, and the fusion order of the four peptide chains is as follows:
(1) antiCD16A.VL~IL-15;
(2) antiCD16A.VH-IL-15Ra~Fc (using knob mutation);
(3) antiCD33.VL VL-CL;
(4) antiCD33.VH-CH1~Fc (using hIgG1, hole mutation, further comprising mutation site H435R).

**Table 2: Cloning designs and construction**

| **Protein number** | **Plasmid number** | **Structural description** | **Sequence number** |
|---|---|---|---|
| QP743745 | QD743 pCHO (DHFR)-antiCD16AFv-IL-15L. R-Fc Knob | anti CD16A VL-IL15 | SEQ ID NO:01 |
| | | anti CD16A VH-IL15Ra-FC(Knob) | SEQ ID NO:02 |
| | QD745-pCHO(GS)-antiCD33-IgG1 (Hole, H435R) | anti CD33 VL-CL | SEQ ID NO:03 |
| | | anti CD33 VH-CH1-CH2-CH3(Hole,H435R) | SEQ ID NO:04 |
| QP43394340 | QD4339: PCHO(DHFR)-antiCD16A.VH-IL-15Ra-FcKnob.Flag(L234A, L235A) | anti CD16A VL-IL15 | SEQ ID NO:01 |
| | | anti CD16A VH-IL15Ra-FC (Knob) (L234A, L235A) | SEQ ID NO:05 |
| | QD4340: PCHO(DHFR)-antiCD33.hIgG1(Hole)(H43 5R).His(L234A, L235A) | anti CD33 VL-CL | SEQ ID NO:03 |
| | | anti CD33 VH-CH1-CH2-CH3(Hole, H435R)(L234A, L235A) | SEQ ID NO:06 |
| QP42914285 | QD4291-pCHO(GS)-antiCD16A-FC-Hole(hIgG1)(H435R)(L234 A, L235A) | anti CD16A VL-CL | SEQ ID NO:07 |
| | | antiCD16A VH-CH1-FC-Hole(hIgG1)(H435R)(L234A, L235A): | SEQ ID NO:08 |
| | QD4285-pCHO(DHFR)-AntiCD33 -IL-15R.L-FC(Knob)(hIgG1) (S228P, F234A, L235A | Anti CD33 VH-IL15Ra-FC(Knob)(hlgGl) (L234A, L235A): | SEQ ID NO:09 |
| | | Anti CD33 VL-IL15 | SEQ ID NO:10 |
| QP7433463 | QD743 pCHO (DHFR)-antiCD16AFv-IL-15L. R-Fc Knob | anti CD16A VL-IL15 | SEQ ID NO:01 |
| | | anti CD16A VH-IL15Ra-FC(Knob) | SEQ ID NO:02 |
| | QD3463-pCHO(GS)-antiCD38-IgG1(Hole)K-His | anti CD38 VL-CL | SEQ ID NO:11 |
| | | anti CD38 VH-CH1-CH2-CH3(Hole, H435R) | SEQ ID NO:12 |
| QP7434264 | QD743 pCHO (DHFR)-antiCD16AFv-IL-15L. R-Fc Knob | anti CD16A VL-IL15 | SEQ ID NO:01 |
| | | anti CD16A VH-IL15Ra-FC(Knob) | SEQ ID NO:02 |
| | QD4264-pCHO(GS)-antiCSl-IgGl(Hole, H435R) | anti CS1 VL-CL | SEQ ID NO:13 |
| | | anti CS1 VH-CH1-CH2-CH3(Hole, H435R) | SEQ ID NO:14 |
| QP43394341 | QD4339: PCHO(DHFR)-antiCD 16A.VH-IL-15Ra-FcKnob.Flag(L234A, L235A) | anti CD16A VL-IL15 | SEQ ID NO:01 |
| | | anti CD16A VH-IL15Ra-FC (Knob) (L234A, L235A) | SEQ ID NO:05 |
| | QD4341:pCHO(GS)-antiCD38-IgG1(Hole)K-His.NEW (L234A, L235A) | anti CD38 VL-CL | SEQ ID NO:11 |
| | | anti CD38 VH-CH1-CH2-CH3(Hole, H435R)(L234A, L235A) | SEQ ID NO:15 |
| QP43394342 | QD4339: PCHO(DHFR)-antiCD 16A.VH-IL-15Ra-FcKnob.Flag(L234A, L235A) | anti CD16A VL-IL15 | SEQ ID NO:01 |
| | | anti CD16A VH-IL15Ra-FC (Knob) (L234A, L235A) | SEQ ID NO:05 |
| | QD4342: pCHO(GS)-antiCS1-IgG1(Hole,H43SR) (L234A, L235A) | anti CS1 VL-CL | SEQ ID NO:13 |
| | | anti CS1 VH-CH1-CH2-CH3(Hole, H435R)(L234A, L235A) | SEQ ID NO:16 |

**Table 3: Cloning designs and construction**

| **Expression and Purification** | **Plasmid** | **Structural description** | |
|---|---|---|---|
| QP42664269 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4269-pCHO(DHFR)-Anti CD33-antiCD16A-IL15L.R-FC(Knob)(hIgG1) (L234A,L235A) | Anti CD33 VL-CL; CD33 VH-IL15Ra-FC(Knob)(hlgG1) (L234A, L235A): | SEQ ID NO:40 |
| | | Anti CD16A VH-Anti CD33 VL-IL15 | SEQ ID NO:41 |
| QP42664301 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4301-pCHO(DHFR)-Anti CD16A-antiCD33-IL15R.L-G1Fc(Knob)(L234A,L235A) | Anti CD16A VH-antiCD33 VL-IL15RaG1Fc(Knob)(L234A,L235A) | SEQ ID NO:42 |
| | | anti CD33 VH-Anti CD16A VL-IL15 | SEQ ID NO:43 |
| QP42664304 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4304-pCHO(DHFR)-Anti CD16A-antiCD33-IL15R.L-G1Fc(Knob)(L234A,L235A) | antiCD33 VL-anti CD16A VH-IL15 | SEQ ID NO:44 |
| | | antiCD16A VL-antiCD33 VH-IL15RaG1Fc(Knob)(L234A,L235A) | SEQ ID NO:45 |
| QP42664307 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4307-pCHO(DHFR)-antiCD33 -antiCD16A-IL15RaG1Fc(Knob)(L234A,L235A) | antiCD33 VL-antiCD16A VH-IL15RaG1Fc(Knob)(L234A,L235A) | SEQ ID NO:46 |
| | | antiCD16A VL-antiCD33 VH-IL15 | SEQ ID NO:47 |
| QP42714274 | QD4271-pCHO(GS)-Anti CD38(HOLE)(hlgG1) (L234A,L235A)(H435R) | anti-CD38 VL-CL: | SEQ ID NO:48 |
| | | Anti CD38 VH-H-Hole(hlgG1)(H435R)(L234A,L235A): | SEQ ID NO:49 |
| | QD4274-pCHO(DHFR)-Anti CD16A-anti CS1-IL15R.L-fc(Knob)(hIgG1) (L234A,L235A) | Anti CS1 VH-antiCD16AVL-IL15Ra-FC(Knob)(hIgG1) (L234A,L235A): | SEQ ID NO:50 |
| | | anti CD16A VH- anti CS1 VL-IL15: | SEQ ID NO:51 |
| QP42714310 | QD4271-pCHO(GS)-Anti CD38(HOLE)(hlgG1) (L234A,L235A)(H435R) | anti-CD38 VL-CL: | SEQ ID NO:48 |
| | | Anti CD38 VH-H-Hole(hlgG1)(H435R)(L234A,L235A): | SEQ ID NO:49 |
| | QD4310-pCHO(DHFR)-AntiCD16A - antiCS1-IL15Ra-FC(Knob)(hIgG1) (L234A,L235A): | AntiCD16A VH- antiCS1 VL -IL15Ra-FC(Knob)(hIgG1) (L234A,L235A): | SEQ ID NO:52 |
| | | AntiCS1 VH-AntiCD16A VL-IL15: | SEQ ID NO:53 |
| QP42714313 | QD4271-pCHO(GS)-Anti CD38(HOLE)(hlgG1) (L234A,L235A)(H435R) | anti-CD38 VL-CL: | SEQ ID NO:48 |
| | | Anti CD38 VH-H-Hole(hlgG1)(H435R)(L234A,L235A): | SEQ ID NO:49 |
| | QD4313-pCHO(DHFR)-AntiCD16A -Anti CS1 -IL15R.L-FC(Knob)(hIgG1) (L234A,L235A) | AntiCD16A VL-Anti CS1 VH-IL15Ra-FC(Knob)(hIgG1) (L234A,L235A): | SEQ ID NO:54 |
| | | anti CS1 VL-anti CD16A VH- IL15: | SEQ ID NO:55 |
| QP42714316 | QD4271-pCHO(GS)-Anti CD38(HOLE)(hlgG1) (L234A,L235A)(H435R) | anti-CD38 VL-CL: | SEQ ID NO:48 |
| | | Anti CD38 VH-H-Hole(hlgG1)(H435R)(L234A,L235A): | SEQ ID NO:49 |
| | QD4316-pCHO(DHFR)-antiCS1-antiCD16A-IL15R.L-FC(Knob)(hIgG1) (L234A,L235A) | antiCS1 VL-antiCD16A VH -IL15Ra-FC(Knob)(hlgG1) (L234A,L235A): | SEQ ID NO:56 |
| | | AntiCD16A VL-Anti CS1 VH -IL15: | SEQ ID NO:57 |
| QP42764279 | QD4276-pCHO(GS)-Anti CS1 (hIgG1)(H435R)(L234A,L235A) | anti CS1 VL-CL | SEQ ID NO:58 |
| | | Anti CS1 VH-H-Hole(hIgG1)(H435R)(L234A,L235A): | SEQ ID NO:59 |
| | QD4279-pCHO(DHFR)-anti CD16A - antiCD38 - IL15R.LFC(Knob)(H435R)(L234 A,L235A) | Anti CD38 VH-antiCD16A VL-IL15Ra-FC (Knob) (H435R) (L234A, L235A) | SEQ ID NO:60 |
| | | anti CD16A VH- antiCD38 VL-IL15: | SEQ ID NO:61 |
| QP42764319 | QD4276-pCHO(GS)-Anti CS1 (hIgG1)(H435R)(L234A,L235A) | anti CS1 VL-CL | SEQ ID NO:58 |
| | | Anti CS1 VH-H-Hole(hIgG1)(H435R)(L234A,L235A): | SEQ ID NO:59 |
| | QD4319-pCHO(DHFR)-AntiCDI 6A-antiCD38-IL15Ra-FC(Knob)(hIgG1) (L234A,L235A) | AntiCD16A VH- antiCD38 VL-IL15Ra-FC(Knob)(hIgG1) (L234A,L235A): | SEQ ID NO:62 |
| | | AntiCD38 VH-AntiCD16A VL -IL15: | SEQ ID NO:63 |
| QP42764322 | | anti CS1 VL-CL | SEQ ID NO:58 |
| | QD4276-pCHO(GS)-Anti CS1 (hIgG1)(H435R)(L234A,L235A) | Anti CS1 VH-H-Hole(hIgG1)(H435R)(L234A,L235A): | SEQ ID NO:59 |
| | QD4322-pCHO(DHFR)-AntiCD16A- Anti CD38-IL15R.L-fc(Knob)(hIgG1) (L234A,L235A) | AntiCD16A VL- Anti CD38 VH-IL15Ra-FC(Knob)(hIgGl) (L234A,L235A): | SEQ ID NO:64 |
| | | AntiCD38 VL-antiCD16A VH- IL15: | SEQ ID NO:65 |
| QP42764325 | QD4276-pCHO(GS)-Anti CS1 (hIgG1)(H435R)(L234A,L235A) | anti CS1 VL-CL | SEQ ID NO:58 |
| | | Anti CS1 VH-H-Hole(hIgG1)(H435R)(L234A,L235A): | SEQ ID NO:59 |
| | QD4325-pCHO(DHFR)-AntiCD38-antiCD16A-IL15R.L-FC(Knob)(hIgG1) (L234A,L235A) | AntiCD38 VL-antiCD16A VH -IL15Ra-FC(Knob)(hIgG1) (L234A,L235A): | SEQ ID NO:66 |
| | | antiCD16A VL- Anti CD38 VH -IL15: | SEQ ID NO:67 |
| QP42824285 | QD4282-pCHO(GS)-antiCD16A - Anti CD33 FC-Hole(hIgG1)(H435R)(L234A,L23 5A) | Anti CD33 VH-antiCD16A VL-CL | SEQ ID NO:68 |
| | | antiCD16A VH-Anti CD33 VL-CH1-FC-Hole(hIgG1)(H435R)(L234A,L235A): | SEQ ID NO:69 |
| | QD4285-pCHO(DHFR)-AntiCD33 -IL15R.L-FC(Knob)(hIgG1) (S228P,F234A,L235A | AntiCD33 VH-IL15Ra-FC(Knob)(hlgGl) (S228P,F234A,L235A): | SEQ ID NO:70 |
| | | Anti CD33 VL-IL15 | SEQ ID NO:71 |
| QP42854328 | QD4328-pCHO(DHFR)-antiCD33-antiCD 16A - Hole(hIgG1)(H435R)(L234A,L23 5A) | Anti CD16A VH-antiCD33 VL-CL | SEQ ID NO:72 |
| | | antiCD33 VH-antiCD16A VL-H-Hole(hIgG1)(H435R)(L234A,L235A): | SEQ ID NO:73 |
| | QD4285-pCHO(DHFR)-AntiCD33 -IL15R.L-FC(Knob)(hIgG1) (S228P,F234A,L235A | AntiCD33 VH-IL15Ra-FC(Knob)(hlgGl) (S228P,F234A,L235A): | SEQ ID NO:70 |
| | | Anti CD33 VL-IL15 | SEQ ID NO:71 |
| QP42854331 | QD4331-pCHO(GS)-anti CD16A-antiCD33 -FC-Hole(hIgG1)(H435R)(L234A,L23 5A) | antiCD33 VL-anti CD16A VH-CL | SEQ ID NO:74 |
| | | anti CD16A VL-antiCD33 VH-CH1-FC-Hole(hIgG1)(H435R)(L234A,L235A): | SEQ ID NO:75 |
| | QD4285-pCHO(DHFR)-AntiCD33 -IL15R.L-FC(Knob)(hIgG1) (S228P,F234A,L235A | AntiCD33 VH-IL15Ra-FC(Knob)(hIgG1) (S228P,F234A,L235A): | SEQ ID NO:70 |
| | | Anti CD33 VL-IL15 | SEQ ID NO:71 |
| QP42854334 | QD4334-pCHO(DHFR)-antiCD33-antiCD 16A - Hole(hIgG1)(H435R)(L34A,L235 A) | AntiCD16A VL-antiCD33 VH-CL | SEQ ID NO:76 |
| | | antiCD33 VL-antiCD16A VH-CH1-FC-Hole(hIgG1)(H435R)(L34A,L235A): | SEQ ID NO:77 |
| | QD4285-pCHO(DHFR)-AntiCD33 -IL15R.L-FC(Knob)(hIgG1) (S228P,F234A,L235A | AntiCD33 VH-IL15Ra-FC(Knob)(hIgG1) (S228P,F234A,L235A): | SEQ ID NO:70 |
| | | Anti CD33 VL-IL15 | SEQ ID NO:71 |
| QP42884285 | QD4288-pCHO(DHFR)-antiCD33-antiCD 16A - Hole(hIgG1)(H435R)(L234A,L23 5A) | antiCD33 VL-anti CD16A VL-CL | SEQ ID NO:78 |
| | | antiCD33 VH-antiCD16A VH-CH1-FC-Hole(hIgG1)(H435R)(L234A,L235A): | SEQ ID NO:79 |
| | QD4285-pCHO(DHFR)-AntiCD33 -IL15R.L-FC(Knob)(hIgG1) (S228P,F234A,L235A | Anti CD33 VH-IL15Ra-FC(Knob)(hlgGl) (L234A, L235A): | SEQ ID NO:70 |
| | | Anti CD33 VL-IL15 | SEQ ID NO:71 |
| QP42664400 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4400-pCHO(DHFR)-IL15L.R-Anti CD16a-FC(Knob)(hIgG1) (L234A,L235A) | Anti CD16a VL-IL15Ra-FC(Knob)(hIgG1) (L234A,L235A): | SEQ ID NO:80 |
| | | IL15-Anti CD16a VH | SEQ ID NO:81 |
| QP42664403 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4403-pCHO(DHFR)-IL15L.R-Anti CD16a-FC(Knob)G1FC(L234A,L235A) | antiCD16A VH-IL15Ra-G1Fc(Knob)(L234A,L235A) | SEQ ID NO:82 |
| | | IL15-anti CD16A VL | SEQ ID NO:83 |
| QP42664406 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4406-pCHO(DHFR)-IL15L.R-Anti CD16-FC(Knob)(hIgG1) (L234A,L235A) | IL15Ra-Anti CD16A VH--FC(Knob)(hIgG1) (L234A,L235A): | SEQ ID NO:84 |
| | | Anti CD16 VL-IL15 | SEQ ID NO:85 |
| QP42664409 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4409-pCHO(DHFR)-IL15L.R-Anti CD16-G1Fc(Knob)(L234A,L235A) | IL15Ra-antiCD16 VL-G1 Fc(Knob)(L234A,L235A) | SEQ ID NO:86 |
| | | anti CD16 VH-IL15 | SEQ ID NO:87 |
| QP42AA4412 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4412-pCHO(DHFR)-IL15L.R-Anti CD16-FC(Knob)(hIgG1) (L234A,L235A) | Anti CD16a VL-IL15Ra | SEQ ID NO:88 |
| | | IL15-Anti CD16a VH-FC(Knob)(hlgG1) (L234A,L235A): | SEQ ID NO:89 |
| QP42664415 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4415-pCHO(DHFR)-IL15L.R-Anti CD16A-G1Fc(Knob)(L234A,L235A) | antiCD16A VH-IL15Ra | SEQ ID NO:90 |
| | | IL15-anti CD16A VL-G1Fc(Knob)(L234A,L235A) | SEQ ID NO:91 |
| QP42AA4418 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4418-pCHO(DHFR)-IL15L.R-Anti CD16A-FC(Knob)(hIgG1) (L234A,L235A) | IL15Ra-Anti CD16A VH | SEQ ID NO:92 |
| | | Anti CD16 VL-IL15--FC(Knob)(hIgG1) (L234A,L235A): | SEQ ID NO:93 |
| QP42664421 | QD4266-pCHO(GS)-Anti-CD33 Hole(hIgG1)(H435R)(L234A,L23 5A) | anti-CD33 VL-CL | SEQ ID NO:38 |
| | | Anti-CD33 VH-H-Hole(hIgG1)(H435R)(L234A,L235A): | SEQ ID NO:39 |
| | QD4421-pCHO(DHFR)-IL15L.R-Anti CD16A-G1Fc(Knob)(L234A,L235A) | IL15Ra-antiCD16 VL | SEQ ID NO:94 |
| | | anti CD16 VH-IL15-GIFc(Knob)(L234A,L235A) | SEQ ID NO:95 |

The sequence of SEQ ID NO: 01 is as follows:
MDMRVPAOLLGLLLLWFPGSRCSYVLTQPSSVSVAPGQTATlSCGGHNIGSKNVHWYQQRPGQS PVLVIYQDNKRPSGIPERFSGSNSGNTATLTISGTQAMDEADYYCQVWDNYSVLFGGGTKLTVLGGG GSGGGGSGGGGSNWVNVISDLKKIEDLIQSMHIDATLYTESDVHPSCKVTAMKFLLELQVISCESGD ASIHDTVENLIILANNSLSSNGNVTESKECEELEEKNIKEFLQSFVHIVQMFINTS*. Among them, the dashed line refers to the VL sequence of anti CD 16A, and the wavy line refers to the IL-15 sequence.

The sequence of SEQ ID NO: 02 is as follows:
MEFGLSWLFLVAILKGVQCQVQLVQSGAEVKKPGESLKVSCKASGYTFTSYYMHWVRQAPGQ GLEWMGIINPSGGSTSYAQKFQGRVTMTRDTSTSTVYMELSSLRSEDTAVYYCARGSAYYYDFADY WGQGTLUTVSSGGGGSGGGGSGGGGSITCPPPMSVEHADIWVKSYSLYRERYICNSGFKRKAGTCS LTECVLNKATNVAHWTTPSLKCIRGGGGSEPKSSDKTHTCPPCPAPELLGGPSVFLFPPKPKDTLMISR TPEVTCVVVDVSHEDPEVKFNWYVDGVEVHNAKTKPREEQYNSTYRWSVLTVLHQDWLNGKEY KCKVSNKALPAPIEKTISKAKGQPREPQVYTLPPSRDELTKNQVSLWCLVKGFYPSDIAVEWESNGQP ENNYKTTPPVLDSDGSFFLYSKLTVDKSRWQQGNVFSCSVMHEALHNHYTQKSLSLSPGK. Among them, the dashed line refers to the VH sequence of anti CD 16A, the wavy line refers to the IL-15Ra sequence, and the double underline refers to Fc (Knob mutation).

The sequence of SEQ ID NO: 03 is as follows:
MDMRVPAQLLGLLLLWFPGSRCDIVMTQSPDSLTVSLGERTTINCKSSQSVLDSSTNKNSLAWY QQKPGQPPKLLLSWEASTRESGIPDRFSGSGSGTDFTLTIDSPQPEDSATYYCQQSAHFPITFGQGTRLEI KRTVAAPSVFIFPPSDEQLKSGTASWCLLNNFYPREAKVQWKVDNALQSGNSQESVTEQDSKDSTY SLSSTLTLSKADYEKHKVYACEVTHQGLSSPVTKSFNRGEC*. Among them, the portion outside the underline is the light chain sequence of antiCD33, and the dashed line refers to the VL sequence.

The sequence of SEQ ID NO: 04 is as follows:
MEFGLSWLFLVAILKGVQCQVQLVQSGAEVKKPGESVKVSCKASGYTFTNYGMNWVKQAPGO GLEWMGWINTYTGEPTYADKFQGRVTMTTDTSTSTAYMEIRNLGGDDTAVYYCARWSWSDGYYV YFDYWGQGTSVTVSSASTKGPSVFPLAPSSKSTSGGTAALGCLUKDYFPEPVTVSWNSGALTSGVHT FPAVLQSSGLYSLSSVVTVPSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSREEMTKNQVSLSCAVKG FYPSDIAVEWESNGQPENNYKTTPPVLDSDGSFFLUSKLTVDKSRWQQGNVFSCSVMHEALHN**R**YT QKSLSLSPGK. Among them, the portion outside the underline is the heavy chain sequence of antiCD33, and the dashed line refers to the VH sequence.

The following sequences are also provided:
antiCD16A VL SEQ ID NO:96 
SYVLTQPSSVSVAPGQTATISCGGHNIGSKNVHWYQQRPGQSPVLVIYQDNKRPSGIPERFSGSNS
GNTATLTISGTQAMDEADYYCQVWDNYSVLFGGGTKLTVL
antiCD16A VH SEQ ID NO:97 
QVQLVQSGAEVKKPGESLKVSCKASGYTFTSYYMHWVRQAPGQGLEWMGIINPSGGSTSYAQ
KFQGRVTMTRDTSTSTVYMELSSLRSEDTAVYYCARGSAYYYDFADYWGQGTLVTVSS
antiCD38VL SEQIDNO:98
antiCD38 VH SEQ ID NO:99
antiCS1 VL SEQ ID NO:100 
DIQMTQSPSSLSASVGDRVTITCKASQDVGIAVAWYQQKPGKVPKLLIYWASTRHTGVPDRFSG
SGSGTDFTLTISSLQPEDVATYYCQQYSSYPYTFGQGTKVEIK
antiCS1 VH SEQ ID NO:101 
EVQLVESGGGLVQPGGSLRLSCAASGFDFSRYWMSWVRQAPGKGLEWIGEINPDSSTINYAPSL
KDKFIISRDNAKNSLYLQMNSLRAEDTAVYYCARPDGNYWYFDVWGQGTLVTVSS
antiCD33 VL SEQ ID NO:102 
DIVMTQSPDSLTVSLGERTTINCKSSQSVLDSSTNKNSLAWYQQKPGQPPKLLLSWASTRESGIP
DRFSGSGSGTDFTLTIDSPQPEDSATYYCQQSAHFPITFGQGTRLEIK
antiCD33 VH SEQ ID NO:103 
QVQLVQSGAEVKKPGESVKVSCKASGYTFTNYGMNWVKQAPGQGLEWMGWINTYTGEPTYA
DKFQGRVTMTTDTSTSTAYMEIRNLGGDDTAVYYCARWSWSDGYYVYFDYWGQGTSVTVSS
IL-15 SEQ ID NO:104 
NWVNVISDLKKIEDLIQSMHIDATLYTESDVHPSCKVTAMKCFLLELQVISLESGDASIHDTVEN
LIILANNSLSSNGNVTESGCKECEELEEKNIKEFLQSFVHIVQMFINTS
IL-15(L52C) SEQ ID NO:105 
NWVNVISDLKKIEDLIQSMHIDATLYTESDVHPSCKVTAMKCFLLELQVISCESGDASIHDTVEN
LIILANNSLSSNGNVTESGCKECEELEEKNIKEFLQSFVHIVQMFINTS
IL-15Ra ECD SEQ ID NO:106 
ITCPPPMSVEHADIWVKSYSLYSRERYICNSGFKRKAGTSSLTECVLNKATNVAHWTTPSLKCIR
DPALVHQRPAPPSTVTTAGVTPQPESLSPSGKEPAASSPSSNNTAATTAAIVPGSQLMPSKSPSTGTTEIS
SHESSHGTPSQTTAKNWELTASASHQPPGVYPQGHSDTT
IL-15Ra Sushi 65 SEQ ID NO:107
   ITCPPPMSVEHADIWVKSYSLYSRERYICNSGFKRKAGTSSLTECVLNKATNVAHWTTPSLKCIR
IL-15Ra Sushi 73 SEQ ID NO:108 
ITCPPPMSVEHADIWVKSYSLYSRERYICNSGFKRKAGTSSLTECVLNKATNVAHWTTPSLKCIR

DPALVHQR
IL-15Ra Sushi 77 SEQ ID NO:109 
ITCPPPMSVEHADIWVKSYSLYSRERYICNSGFKRKAGTSSLTECVLNKATNVAHWTTPSLKCIR

DPALVHQRPAPP
IL-15Ra Sushi 86 SEQ ID NO:110 
ITCPPPMSVEHADIWVKSYSLYSRERYICNSGFKRKAGTSSLTECVLNKATNVAHWTTPSLKCIR
DPALVHQRPAPPSTVTTAGVT
IL-15Ra Sushi 102 SEQ ID NO:111 
ITCPPPMSVEHADIWVKSYSLYSRERYICNSGFKRKAGTSSLTECVLNKATNVAHWTTPSLKCIR
DPALVHQRPAPPSTVTTAGVTPQPESLSPSGKEPAAS
IL-15Ra-sushi(S40C): SEQ ID NO:112 ITCPPPMSVEHADIWVKSYSLYSRERYICNSGFKRKAGTCSLTECVLNKATNVAHWTTPSLKCIR.

All the sequences of IL-15 or IL-15Ra mentioned above can be used to construct the fusion protein of the present invention.

As shown in Table 4, the control antibodies, antiCD16a antibody, antiCD33 antibody, Elotuzumab, Daratuumab, TAA/CD16A bispecific antibody were designed and constructed, and CD33/CD16A/IL-15 control molecules such as GTB-3550 were constructed according to patent literature US20200148737A1.

**Table 4 Design and construction of control antibody clones**

| Target | Protein number | Plasmid number | Sequence number |
|---|---|---|---|
| CD33 antibody | QP41154116 | QD4115(CD33 VL)-pQDK | SEQ ID NO:17 |
| | | QD4116(CD33 VH)-pQDH(IgGl) | SEQ ID NO:18 |
| CS1 antibody | QP34483449 | QD3448-pQD-anti-CS1.IgG1.gb | SEQ ID NO:19 |
| | | QD3449-pQDK-ANTI-CS1.gb | SEQ ID NO:20 |
| CD38 antibody | QP34503451 | QD3450-pQDH-ANTI-CD38.IgG1.gb | SEQ ID NO:21 |
| | | QD3451-pQDK-ANTI-CD38.gb | SEQ ID NO:22 |
| CD16A abtibody | QP43354336 | QD4336-PQDH(BsmbI/NotI)-CD16A-VH-CHl-FC(IgGl-L234A, L235A) | SEQ ID NO:23 |
| | | QD4335-PQDK(Bsmbi)-CD16A-VL-CL | SEQ ID NO:24 |
| CD16A/CD33 bispecific antibody | QP4115777778 | QD778:pQD-CD16A VH-VL-FC(Knob)-flag | SEQ ID NO:25 |
| | | QD777:pQD-CD33 VH-CHl-FC(Hole-H435R)-his | SEQ ID NO:26 |
| | | QD4115(CD33 VL)-pQDK | SEQ ID NO:27 |
| CD16A/CD33 bispecific antibody (LALA) | QP41154367436 8 | QD4368: pQD-CD 16A VH-VL-FC(Knob)-flag (L234A, L235A) | SEQ ID NO:28 |
| | | QD4367: pQD-CD33 VH-CHl-FC(Hole-H435R)-his (L234A, L235A) | SEQ ID NO:29 |
| | | QD4115(CD33 VL)-pQDK | SEQ ID NO:30 |
| 161533 trispecific killer engager (TriKE), GTB-3550 | GTB-3550 (US2020014873 7A1_1) | QD877 | SEQ ID NO:31 |
| IL15/IL15Ra-FC | QP33123313 | QD3312 | SEQ ID NO:32 |
| | | QD3313 | SEQ ID NO:33 |
| CD16A/CS1 bispecific antibody (LALA) | QP34494456436 8 | QD4368: pQD-CD 16A VH-VL-FC(Knob)-flag (L234A, L235A) | SEQ ID NO:34 |
| | | QD4456-pQD-CSl VH-CHl-FC(Hole-H435R)-his (QD777-L234A, L235A) | SEQ ID NO:35 |
| | | QD3449-pQDK-ANTI-CS1.gb | SEQ ID NO:20 |
| CD16A/CD38 bispecific antibody (LALA) | QP34514457436 8 | QD4368: pQD-CD 16A VH-VL-FC(Knob)-flag (L234A, L235A) | SEQ ID NO:36 |
| | | QD4457-pQD-CD38 VH-CHl-FC(Hole-H435R)-his (QD777-L234A, L235A) | SEQ ID NO:37 |
| | | QD3451-pQDK-ANTI-CD38.gb | SEQ ID NO:22 |

### Transient expression of target molecules:

ExpiCHO-S cells were inoculated into FortiCHO medium (Gibco, A1148301) and 8mM GlutaMax was added, then cultured at 37 °C, 120rpm, and 8% CO₂. The day before transfection, the cell density of Expi CHO-S was adjusted to 3*10E6/mL, placed in a shaking table, and cultured at 37°C, 120rpm and 8% CO₂. On the day of transfection, cells were taken and counted, the cell density was diluted to 6*10E6/ml, 40ml per bottle, and placed in a 125ml shake flask. 20µg of the co-transfection plasmids was taken and mixed with 4.8mL Opti MEM, and 120ul Polyplus-Fecto PRO transfection reagent was added. The DNA and the transfection reagent were mixed evenly, then it was placed at room temperature for 10min. The mixture was slowly added into the cells, mixed evenly, and placed in a shaker for culture. During the culture, 2mL Feed PFF05 (OPM, F81279-001) and 1mL 30% glucose solution were supplemented to each bottle on the 1st, 4th, 6th and 8th days respectively. On the first day of transfection, the temperature was reduced to 32 °C and the CO₂ concentration was reduced to 5%. Samples were collected on the 13th day, centrifuged at 8000rpm for 20min, and supernatant was taken for purification.

### Purification of fusion proteins

### Protein A affinity chromatography purification

At least 3CV (actual volume 20 ml) of equilibrium solution was allowed to pass through the column, so as to ensure that the pH and conductivity of the final solution flowing out of the instrument were consistent with the equilibrium solution, and the flow rate was 1 ml/min; the culture supernatant after centrifugation was allowed to pass through the column, 40 ml of sample was loaded, and the flow rate was 0.33 ml/min; at least 3CV (actual volume is 20 ml) of equilibrium solution was allowed to pass through the column, so as to ensure that the pH and conductivity of the final solution flowing out of the instrument were consistent with the equilibrium solution, and the flow rate was 0.33 ml/min; the eluent was allowed to pass through the column, and the elution peaks (PAC-EP) began to be collected when the UV280 increased to 15 mAU; and collection was stopped when the UV280 decreased to 15 mAU, and the flow rate was 1 ml/min. After the sample collection was completed, the PAC-EP was adjusted to neutral with a pH adjustment solution.

### CH1-XL affinity chromatography

The sample treated with Protein A was centrifuged at 8000rpm for 15 min, and the supernatant was collected. At least 3CV (actual volume 20 ml) of equilibrium solution was allowed to pass through the column, so as to ensure that the pH and conductivity of the final solution flowing out of the instrument were consistent with the equilibrium solution, and the flow rate was 1 ml/min; the supernatant after centrifugation was allowed to pass through the column through the sample loading loop, and the flow rate was 0.33 ml/min; at least 3CV (actual volume is 20 ml) of equilibrium solution was allowed to pass through the column, so as to ensure that the pH and conductivity of the final solution flowing out of the instrument were consistent with the equilibrium solution, and the flow rate was 0.33 ml/min; the eluent was allowed to pass through the column, and the elution peaks (PAC-EP) began to be collected when the UV280 increased to 10 mAU; and collection stopped when the UV280 decreased to 10 mAU, and the flow rate was 1 ml/min. After the sample collection was completed, the CH1-EP was adjusted to neutral with a pH adjustment solution.

### Example 2: FACS detection of CD33/CD16A/IL-15 trifunctional fusion protein binding to HL60 cells naturally expressing CD33

Experimental objective: Human promyelocytic leukemia cell line HL60 naturally expresses CD33. The present invention uses FACS to detect the binding of CD33/CD16A/IL-15 trifunctional fusion protein to HL60 cells naturally expressing CD33.

Experimental method: HL60 cells were cultured in a 5% CO₂ incubator at 37 °C. After trypsin digestion, cells were collected and inoculated into 96-well plates at 100,000 cells per well. Then the plate was subjected to blocking on ice for 1 hour with 2% FBS/PBS. The cells were incubated with different concentrations of trifunctional proteins and control protein on ice for 1 hour, washed with PBS for 3 times, and incubated with PE-anti human Fc (1:200 dilution). The cells were washed with PBS for 3 times, and resuspended with 200 µl PBS. The mean fluorescence value was read by FACS and Graphpad prism software was used to analyze the results.

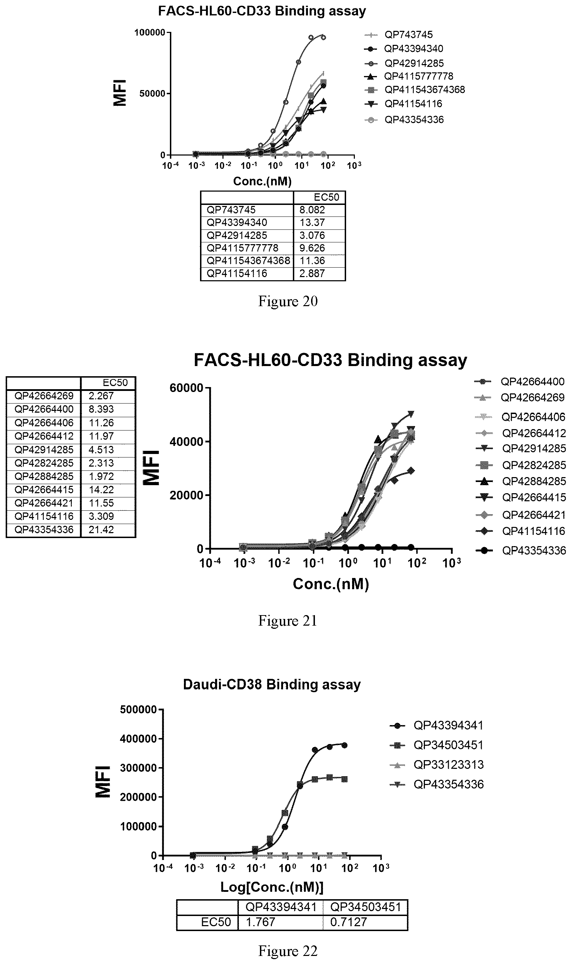

As shown in Figure 20, CD33/CD16A/IL-15 trifunctional fusion protein QP743745 (FC wild-type), QP43394340 (FC with L234A/L235A mutation for eliminating function), QP42914285 (FC with L234A/L235A mutation for eliminating function), CD33/CD16A bispecific antibody QP411577778 (FC wild-type), CD33/CD16A bispecific antibody QP411543674368 (FC with L234A/L235A mutation for eliminating function) and CD33 antibody IgG1wt subtype QP41154116 all bound to HL60 cells naturally expressing CD33.

As shown in Figure 21, both CD33 antibody IgGlwt subtype QP41154116 and CD33/CD16A/IL-15 trifunctional fusion protein bound to HL60 cells naturally expressing CD33.

### Example 3: FACS detection of CD38/CD16A/IL-15 trifunctional fusion protein binding to Daudi cells naturally expressing CD38

Experimental objective: Human Burkitt's lymphoma cells, Daudi cells, naturally express CD38. The present invention uses FACS to detect the binding of CD38/CD16A/IL-15 trifunctional fusion protein to Daudi cells naturally expressing CD38.

Experimental method: Daudi cells were cultured in a 5% CO₂ incubator at 37 °C. After trypsin digestion, cells were collected and inoculated into 96-well plates at 100,000 cells per well. Then the plate was subjected to blocking on ice for 1 hour with 2% FBS/PBS. The cells were incubated with different concentrations of trifunctional proteins and control protein on ice for 1 hour, washed with PBS for 3 times, and incubated with PE-anti human Fc (1:200 dilution). The cells were washed with PBS for 3 times, and resuspended with 200 µl PBS. The mean fluorescence value was read by FACS and Graphpad prism software was used to analyze the results.

As shown in Figure 22, both CD38 antibody IgGlwt subtype QP34503451 and CD38/CD16A/IL-15 trifunctional fusion protein QP43394341 (FC with L234A/L235A mutation eliminating function) bound to Daudi cells naturally expressing CD38 in a concentration dependent manner.

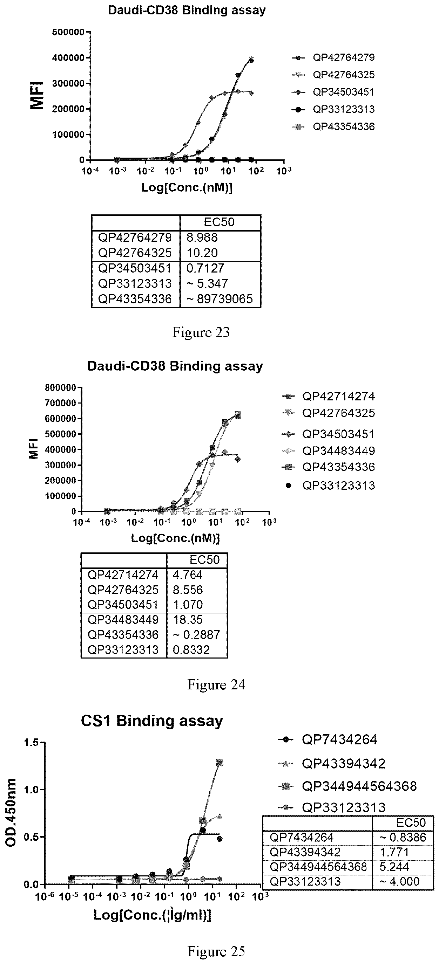

As shown in Figure 23 and Figure 24, both CD38 antibody IgGlwt subtype QP34503451 and CD38/CS1/CD16A/IL-15 trifunctional fusion protein bound to Daudi cells naturally expressing CD38 in a concentration dependent manner.

### Example 4: ELISA detection of the Activity of CS1/CD16A/IL-15 trifunctional fusion protein binding to CS1

**Detection reagents:** Milk (BD, 232100), PBS (Sangon, B548117-0500); HRP anti human IgG (H+L) (jackson, 109-035-088); TMB (Luoyang Baiaotong Experimental Materials Center, C060201); Elisa plate (costa, 9018) 1 × PBS buffer: NaCl 8.00g, KCl 0.20g, Na₂HPO₄ • 12H₂O 2.9g, KH₂PO₄ 0.2g were weighed and added to 800mL ddH₂O for dissolution, after dissolving thoroughly, the volume was determined to 1L, and the pH was adjusted to 7.4, and followed by sterilizing at high temperature for later use. Alternatively, commercial 10× or 20× PBS solution was diluted to 1× PBS buffer for use. Blocking solution: 5g milk was weighed and added into PBS. The blocking solution shall be prepared for current use. Stop solution (1mol/L H₂SO₄): 109mL 98% concentrated H₂SO₄ was slowly added dropwise into 2000mL ddH₂O. After developing with TMB at 37°C for 10min, the plate was placed in a shaker (120rpm), 100 µl/well.

**Experimental steps:** The plate was coated with CS1-Fc at 1µg/ml, 4°C overnight, and washed with PBS for 3 times. 5% milk of blocking solution was added at 200 µL/well and incubated at 37°C for 1h. After blocking, the plate was washed with PBS for 3 times, and sample incubation was performed. The sample was diluted by 5 times at 20 µg/ml, with a total of 7 gradients and the final well was diluted by 100 times, 100 µl/well, mixed evenly, and incubated for 1h. Then the plate was washed with PBST for 3 times. Adding enzyme labeled antibody: incubating HRP-anti human Fab antibody at a dilution ratio of 1:5000, 100 µl/well, mixed evenly, and incubated for 1h, then the plate was washed with PBST for 6 times. Adding substrate chromogenic solution: substrate chromogenic solution TMB was added at 100 µL/well. The plate was placed in a shaker, 200rpm, developing in the dark at 35°C for 10min. Termination: After the development was completed, the stop solution was immediately added at 100 µL/well to terminate the reaction. Detection: The OD value at A450nm was measured on the microplate reader, and the results were analyzed by Graphpad prism software.

As shown in Figure 25, both CS1/CD16A/IL-15 trifunctional fusion protein QP7434262 (FC wildtype) and QP43394342 (FC with L234A/L235A mutation for eliminating function) bound to CS1 protein in a concentration dependent manner.

### Example 5: Mo7e cell proliferation experiment

Mo7e (human giant cell leukemia cell line) cell expresses IL-15R βγ. It is a cytokine growth dependent cell. Previous studies have shown that resting NK and immature T cells express a moderate affinity IL-15R βγ phenotype. The results of the cell proliferation experiment of cytokine IL-15/IL-15Ra on Mo7e (IL-15Rβγ) were consistent with the proliferation experiment on unstimulated PBMCs (Mol Cancer Ther; 11 (6) June 2012). The present invention evaluated the IL-15 activity of multifunctional fusion protein using Mo7e cell proliferation assay. The method and results are as follows:
**Experimental reagent:** Mo7e cells (human giant cell leukemia cell line), purchased from the Cell Resource Center of Institute of Basic Medicine, Chinese Academy of Medical Sciences. Cell Proliferation and Toxicity Test Kit (CCK-8), purchased from Meilunbio, catalog number MA0218; Recombinant human GM-CSF, purchased from Perprotech, catalog number 300-03; Human IgG, purchased from Sigma, catalog number I4506. Other antibodies were prepared internally.

**Experimental method:** Mo7e cells were cultured in RPMI1640 medium containing 10% FBS, 2mM L-glutamine and 8ng/ml GM-CSF in a 5% CO₂ incubator at 37°C. Mo7e cells were collected, centrifuged at 800rpm for 5 minutes to pour out the supernatant, and the cells were washed twice with RPMI1640 medium without GM-CSF. The cells were resuspended with GM-CSF-free RPMI1640 medium and counted, then inoculated into 96-well plates at 2 × 10⁴ cells with 80 µl per well, and cultured in a 5% CO₂ incubator at 37°C for 1 hour. Each drug to be tested was diluted with a 4-fold gradient in the culture medium, evenly mixed with cell suspension at 20 µl per well, and the plate was cultured in a 5% CO₂ incubator at 37°C for 3 days. The CCK-8 reagent was added to the 96-well plate to be tested at 10 µl per well, and incubated in a 5% CO₂ incubator at 37°C for 4 hours. The 96-well plate was taken out and the absorbance value at 450 nm wavelength was detected in the microplate reader.

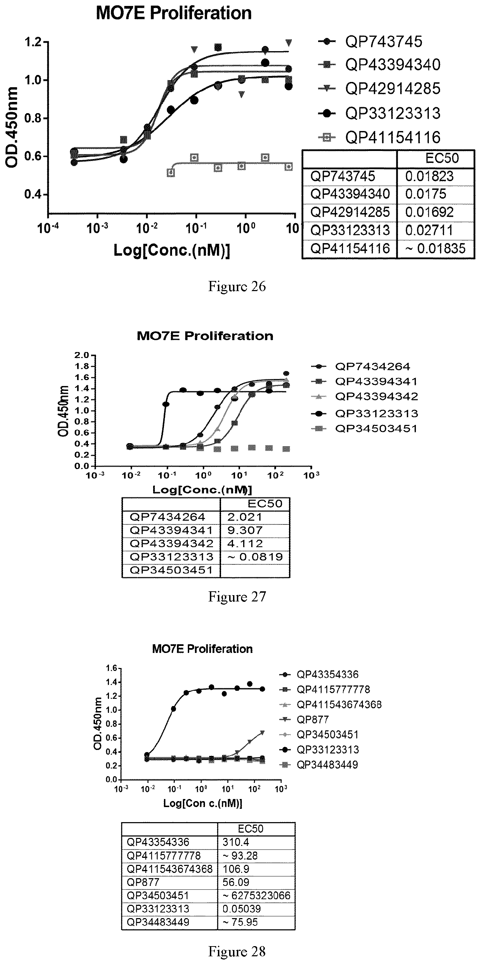

Figures 26, 27, and 28 show that the trifunctional fusion proteins CD33/CD16A/IL-15, CD38/CD16A/IL-15, and CS1/CD16A/IL-15 can all promote the proliferation of MO7E cells, proving that all the molecular form proteins of the trifunctional fusion proteins of the present invention have IL-15 biological activity.

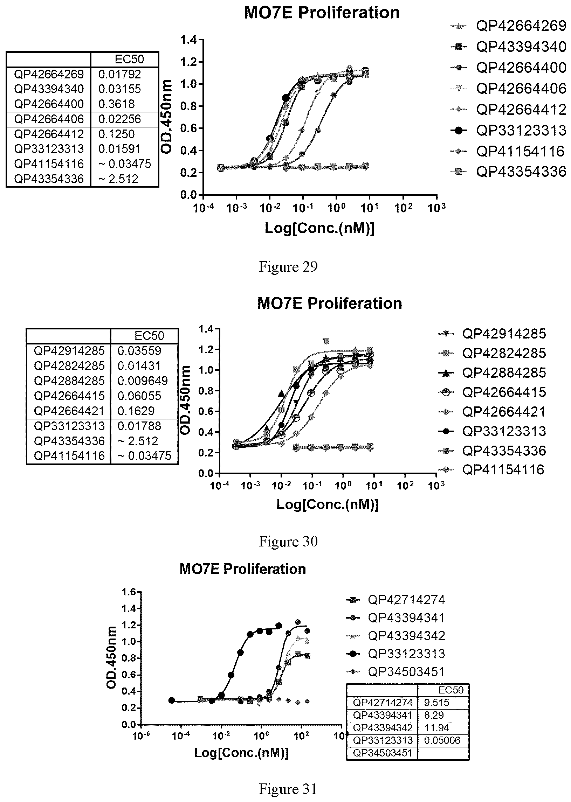

Figure 29, Figure 30, Figure 31 and 32 show that both the trifunctional fusion proteins CD33/CD16A/IL-15 and CD38/CS1/CD16A/IL-15 can promote the proliferation of MO7E cells, proving that all the molecular form proteins of the multifunctional fusion proteins of the present invention have IL-15 biological activity.

### Example 6: C33/CD16A/IL-15 trifunctional fusion protein-mediated ADCC effect

Experimental objective: To detect the CD33/CD16A/IL-15 trifunctional fusion protein through ADCC assay.

Experimental materials: PBMCs were purchased from Shanghai Saili Biotechnology Co., Ltd., HL60 cells were purchased from the cell bank of the Chinese Academy of Sciences, and Cytotox96 non-radioactive cytotoxicity assay kit was purchased from Promega (G1780).

Experimental steps: PBMCs were resuscitated and cells were collected for later use the next day. Preparation of target cells: HL60 cells were digested with trypsin, 1000rpm for 5 minutes. After washed with PBS twice, the cells were inoculated into 96-well plates at 20,000 cells per well, 50 µl per well, and incubated at 5% CO₂, 37°C for 2 hours. Preparation of antibodies: The antibody was diluted with a gradient using culture medium (RPMI1640 containing 10% low-IgG FBS) for 8 concentrations. The above diluted antibody in each concentration was added at 50 µl per well and incubated at 37°C for 15min. Preparation of PBMC: PBMCs resuscitated on the first day were centrifuged, resuspended in culture medium (RPMI1640 containing 10% low-IgG FBS), and were counted. According to the rate of PBMC: Target cell = 20:1 or 10:1, cells were added into the well-plate at 50 µl per well and incubated at 37°C for 4 hours. LDH detection: for the maximum release group and volume correction group, 10 µl lysate was added 45min in advance, and continued to be cultured in the incubator. After culture for 4h, 50 µl of supernatant was absorbed into the ELISA plate, and 50 µl of reagent was added according to the instructions of Cytotox96 non-radioactive cytotoxicity assay kit (Promega, G1780). After reaction in the dark at room temperature for 30 min, 50 µl stop solution was added, and absorbance value at 490nm was read (the reading was completed within 1h after adding stop solution). Calculation: killing percentage = (sample release - target cell spontaneous release - effector cell spontaneous release)/(target cell maximum release - target cell spontaneous release) * 100. Spontaneous release (corresponding to target cells incubated with effector cells in the absence of antibodies) was defined as 0% cytotoxicity, and maximum release (target cells lysed with 1% Triton X-100) was defined as 100% cytotoxicity.

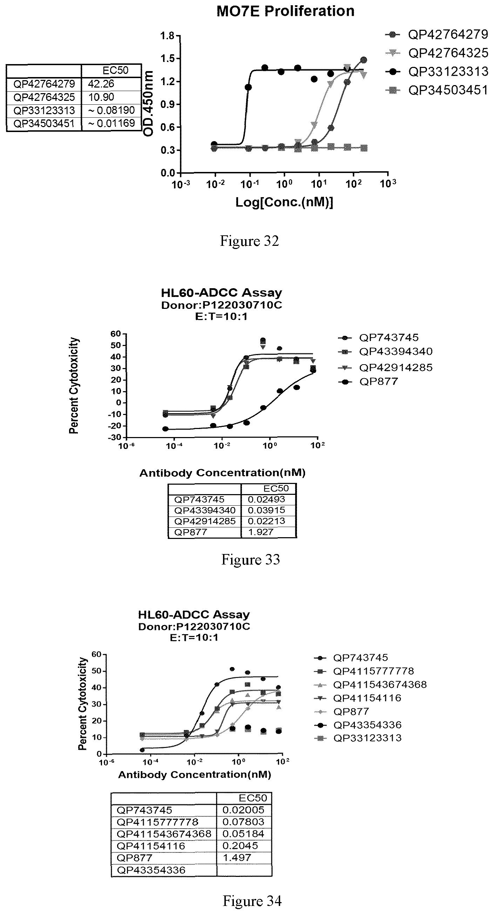

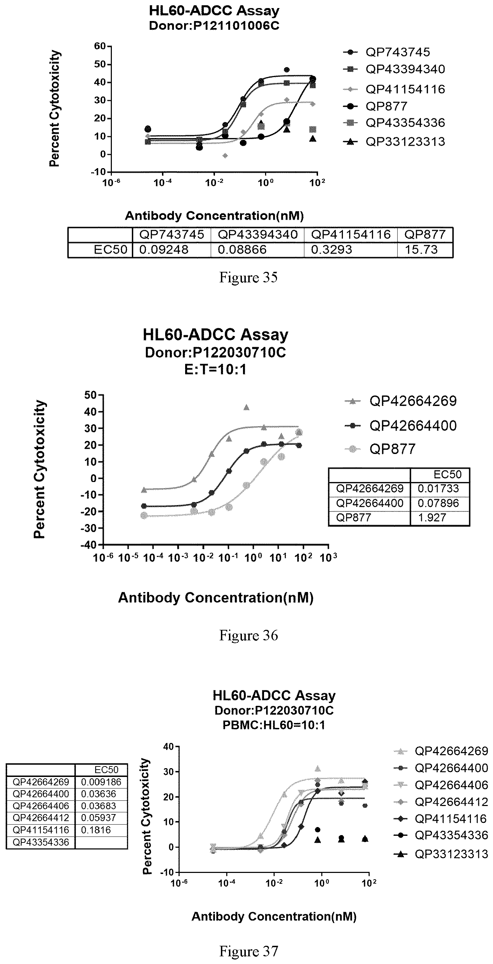

Figure 33 and Figure 34 show the results of the effector target ratio of 10:1, and Figure 35 shows the result of the effector target ratio of 20:1. The results showed that the CD33 antibody IgG1wt subtype QP41154116 mediated NK cell killing of HL60 cells naturally expressing CD33 in a concentration dependent manner. The NK cell killing of HL60 cells mediated by the CD33/CD16A/IL-15 trifunctional fusion protein QP743745 (FC wild-type), QP43394340 (FC with L234A/L235A mutation for eliminating function), QP42914285 (FC with L234A/L235A mutation for eliminating function), and the CD33/CD16A bispecific antibody QP411577778 (FC wild-type), the CD33/CD16A bispecific antibody QP411543674368 (FC with L234A/L235A mutation for eliminating function) was superior to that mediated by the CD33/CD16A/IL-15 trifunctional fusion protein control molecule GT-3550 (QP877) and CD33 antibody IgGlwt subtype QP41154116. CD16A monoclonal antibody QP433354336 and IL-15/IL-15Ra-FC fusion protein QP33123313 showed no ADCC effect.

Figure 36, Figure 37 and Figure 38 show the results of the effector target ratio of 10:1. The results showed that the CD33 antibody IgG1wt subtype QP41154116 mediated NK cell killing of HL60 cells naturally expressing CD33 in a concentration dependent manner, and the NK cell killing of HL60 cells mediated by the CD33/CD16A/IL-15 trifunctional fusion protein (FC with L234A/L235A mutation for eliminating function) was superior to that mediated by the CD33/CD16A/IL-15 trifunctional fusion protein control molecule GT-3550 (QP877) and CD33 antibody IgGlwt subtype QP41154116. CD16A monoclonal antibody QP433354336 and IL-15/IL-15Ra-FC fusion protein QP33123313 showed no ADCC effect.

### Example 7: CD38/CD16A/IL-15 trifunctional fusion protein-mediated ADCC effect

Experimental objective: To detect the CD38/CD16A/IL-15 trifunctional fusion protein through ADCC assay.

Experimental materials: PBMCs were purchased from Shanghai Saili Biotechnology Co., Ltd., Daudi cells were purchased from the cell bank of the Chinese Academy of Sciences, and Cytotox96 non-radioactive cytotoxicity assay kit was purchased from Promega (G1780).

Experimental steps: PBMCs were resuscitated and cells were collected for later use the next day. Preparation of target cells: Daudi cells were digested with trypsin, 1000rpm for 5 minutes. After washed with PBS twice, the cells were inoculated into 96-well plates at 20,000 cells per well, 50 µl per well, and incubated at 5% CO₂, 37°C for 2 hours. Preparation of antibodies: The antibody was diluted with a gradient using culture medium (RPMI1640 containing 10% low-IgG FBS) for 8 concentrations. The above diluted antibody in each concentration was added at 50 µl per well and incubated at 37°C for 15min. Preparation of PBMC: PBMCs resuscitated on the first day were centrifuged, resuspended in culture medium (RPMI1640 containing 10% low-IgG FBS), and were counted. According to tha rate of PBMC: Target cell = 10:1 or 5:1, cells were added into the well-plate at 50 µl per well and incubated at 37°C for 4 hours. LDH detection: for the maximum release group and volume correction group, 10 µl lysate was added 45min in advance, and continued to be cultured in the incubator. After culture for 4h, 50 µl of supernatant was absorbed into the ELISA plate, and 50 µl of reagent was added according to the instructions of Cytotox96 non-radioactive cytotoxicity assay kit (Promega, G1780). After reaction in the dark at room temperature for 30 min, 50 µl stop solution was added, and absorbance value at 490nm was read (the reading was completed within 1h after adding stop solution). Calculation: killing percentage = (sample release - target cell spontaneous release - effector cell spontaneous release)/(target cell maximum release - target cell spontaneous release) * 100. Spontaneous release (corresponding to target cells incubated with effector cells in the absence of antibodies) was defined as 0% cytotoxicity, and maximum release (target cells lysed with 1% Triton X-100) was defined as 100% cytotoxicity.

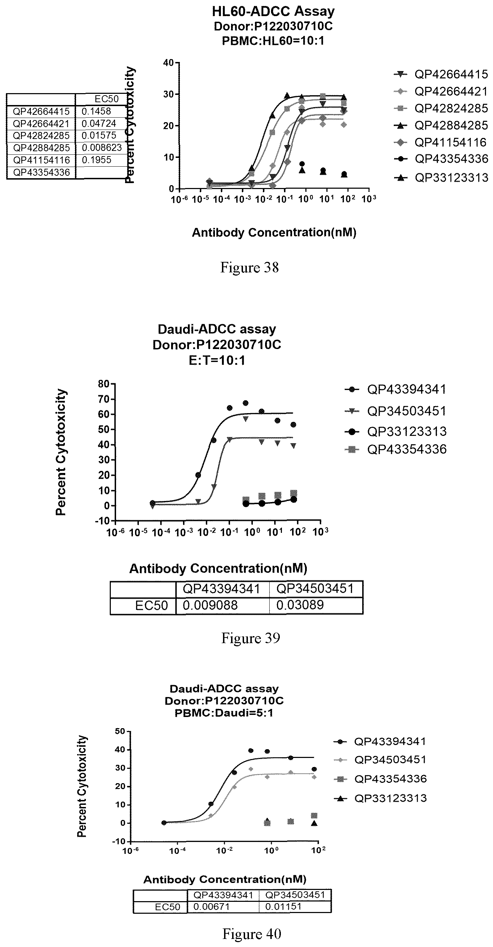

Figure 39 shows the results of the effector target ratio of 10:1, and Figure 40 shows the result of the effector target ratio of 20:1. As shown in the figure, at different effector target ratios, the CD38 antibody IgG1wt subtype QP34503451 showed a concentration dependent effect on NK cell killing of Daudi cells naturally expressing CD38. The NK cell killing of Daudi cells mediated by the CD38/CD16A/IL-15 trifunctional fusion protein QP43394341 (FC with L234A/L235A mutation for eliminating function) was superior to that mediated by the CD38 antibody IgGlwt subtype QP34503451. CD16A monoclonal antibody QP433354336 and IL-15/IL-15Ra-FC fusion protein QP33123313 showed no ADCC effect.

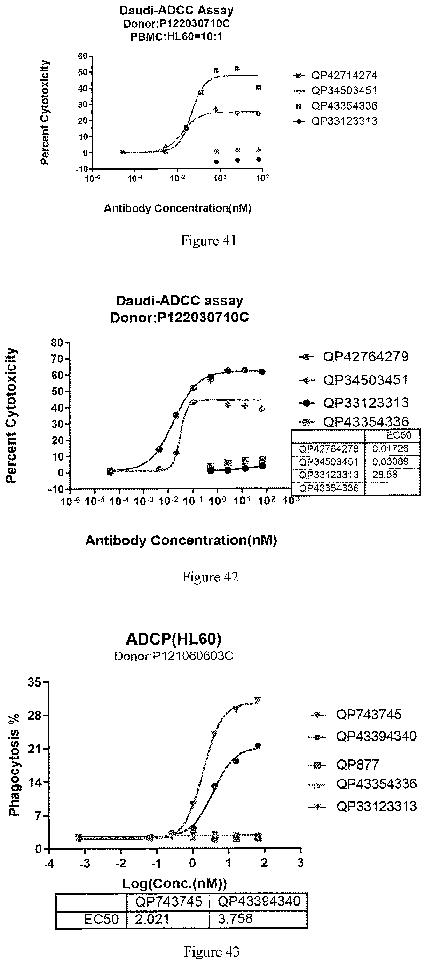

Figure 41 and Figure 42 show the results of the effector target ratio of 10:1. The CD38 antibody IgG1wt subtype QP34503451 showed a concentration dependent effect on NK cell killing of Daudi cells naturally expressing CD38. The NK cell killing of Daudi cells mediated by the CD38/CD16A/IL-15 trifunctional fusion protein QP43394341 (FC with L234A/L235A mutation for eliminating function) was superior to that mediated by the CD38 antibody IgGlwt subtype QP34503451. CD16A monoclonal antibody QP433354336 and IL-15/IL-15Ra-FC fusion protein QP33123313 showed no ADCC effect.

### Example 8: CD33/CD16A/IL-15 trifunctional fusion protein-mediated ADCP effect

Monocytes were separated from peripheral blood mononuclear cells (PBMCs) of healthy individuals, and were added with 50 ng/mL Human Recombinant M-CSF to induce differentiation into macrophages. HL60 cells were labeled with green fluorescent CFSE, and were inoculated into a 96 well plate with macrophages at a ratio of 2:1, and then the plate was added with different concentrations of proteins to be tested. After incubating at 37°C for 2 hours, the reaction was terminated, and then the APC anti-human CD11b antibody was incubated. By reading FACS, the percentage of APC/FITC double positive cells at each concentration of antibody was obtained, which was the percentage of macrophages exhibiting phagocytic behavior.

As shown in Figure 43, the CD33/CD16A/IL-15 trifunctional fusion protein QP743745 (FC wild-type) and QP43394340 (FC with L234A/L235A mutation for eliminating function) both exhibited concentration dependent phagocytosis of HL60 cells by macrophages. CD33/CD16A/IL-15 trifunctional fusion protein control molecule GT-3550 (QP877) showed no ADCP effect.

Although the content of the present invention has been described in detail through the preferred embodiments mentioned above, it should be recognized that the above description should not be considered a limitation of the present invention. After reading the above disclosure, it will be apparent to those skilled in this art that there are various modifications and substitutions to the present invention. Therefore, the scope of protection of the present invention should be limited by the appended claims.

## Claims

1. An antigen targeting, anti-CD16A, and immune effector cell activating trifunctional fusion protein, wherein the antigen comprises a tumor associated antigen (TAA), the trifunctional fusion protein comprises: CD16A binding region that specifically binds to CD16A, TAA binding region that specifically binds to tumor associated antigen (TAA), IL-15/IL-15Rα complex formed by the interaction of IL-15 and IL-15Rα, and Fc domain.

2. The antigen targeting, anti-CD16A, and immune effector cell activating trifunctional fusion protein according to claim 1, wherein the CD16A binding region comprises an anti-CD16A antibody and fragment thereof, preferably, the CD16A binding region is a Fab fragment or Fv fragment targeting CD16A.

3. The antigen targeting, anti-CD16A, and immune effector cell activating trifunctional fusion protein according to claim 1, wherein the TAA binding region comprises an anti-TAA antibody and fragment thereof, preferably, the TAA binding region is a Fab fragment or Fv fragment targeting TAA.

4. The antigen targeting, anti-CD16A, and immune effector cell activating trifunctional fusion protein according to claim 1, wherein the fusion protein further comprises a connecting fragment; and the amino acid sequence of the connecting fragment is preferably composed of several GS repeat sequences.

5. The antigen targeting, anti-CD16A, and immune effector cell activating trifunctional fusion protein according to claim 1, wherein the fusion protein comprises several peptide chains, wherein the IL-15 and IL-15Rα are located on different peptide chains, respectively.

6. The antigen targeting, anti-CD16A, and immune effector cell activating trifunctional fusion protein according to claim 1, wherein the trifunctional fusion protein comprises one TAA binding region.

7. The antigen targeting, anti-CD16A, and immune effector cell activating trifunctional fusion protein according to claim 6, wherein the fusion protein comprises a first monomer and a second monomer; wherein
the first monomer comprises: a TAA binding region and a first Fc chain; the second monomer comprises: the CD16A binding region, the IL-15/IL-15Rα complex and a second Fc chain; the TAA binding region is a Fab fragment targeting TAA; the CD16A binding region is a Fv fragment targeting CD16A;
or,
the first monomer comprises: the CD16A binding region and a first Fc chain; the second monomer comprises: the TAA binding region, the IL-15/IL-15Rα complex and a second Fc chain; the CD16A binding region is a Fab fragment targeting CD16A; the TAA binding region is a Fv fragment targeting TAA;
and,
the first Fc chain and the second Fc chain polymerize to form the Fc domain.

8. The antigen targeting, anti-CD16A, and immune effector cell activating trifunctional fusion protein according to claim 7, wherein the first monomer comprises a first peptide chain and a second peptide chain, the second monomer comprises a third peptide chain and a fourth peptide chain;
the first peptide chain comprises: a TAA binding antibody variable region, an antibody constant region;
the second peptide chain comprises: a TAA binding antibody variable region, an antibody constant region; the constant region of the second peptide chain comprises a first Fc chain;
the third peptide chain comprises: a CD16A antibody variable region, a cytokine, and a second Fc chain;
the fourth peptide chain comprises: a CD16A antibody variable region, a cytokine;
the pairing regions of the TAA binding antibody variable region and the antibody constant region of the first peptide chain, as well as the TAA binding antibody variable region and the constant region of the second peptide chain to form the Fab fragment targeting TAA;
the CD 16A binding antibody variable region of the third peptide chain and the CD16A binding antibody variable region of the fourth peptide chain form the Fv fragment targeting CD16A;
the cytokine of the third peptide chain interacts with the cytokine of the fourth peptide chain to form the IL-15/IL-15Rα complex.

9. The antigen targeting, anti-CD16A, and immune effector cell activating trifunctional fusion protein according to claim 7, wherein the first monomer comprises a first peptide chain and a second peptide chain, the second monomer comprises a third peptide chain and a fourth peptide chain;
the first peptide chain comprises: a CD16A binding antibody variable region, an antibody constant region;
the second peptide chain comprises: a CD16A binding antibody variable region, an antibody constant region; the constant region of the second peptide chain comprises a first Fc chain;
the third peptide chain comprises: a TAA antibody variable region, a cytokine, and a second Fc chain;
the fourth peptide chain comprises: a TAA antibody variable region, a cytokine;
the pairing regions of the CD16A binding antibody variable region and the antibody constant region of the first peptide chain, as well as the CD16A binding antibody variable region and the constant region of the second peptide chain to form the Fab fragment targeting CD16A;
the TAA binding antibody variable region of the third peptide chain and the TAA binding antibody variable region of the fourth peptide chain form the Fv fragment targeting TAA;
the cytokine of the third peptide chain interacts with the cytokine of the fourth peptide chain to form the IL-15/IL-15Rα complex.

10. The antigen targeting, anti-CD16A, and immune effector cell activating trifunctional fusion protein according to claims 8 and 9, wherein:
from the N-terminus to the C-terminus of the peptide chain, the fusion order of the third peptide chain is antibody variable region~cytokine~the second Fc chain, or cytokine-antibody variable region-the second Fc chain;
from the N-terminus to the C-terminus of the peptide chain, the fusion order of the fourth peptide chain is antibody variable region-cytokine; or cytokine-antibody variable region; "∼" represents a connecting fragment;
the antibody variable region is a CD16A binding antibody variable region or a TAA binding antibody variable region.

11. The antigen targeting, anti-CD16A, and immune effector cell activating trifunctional fusion protein according to claim 1, wherein the trifunctional fusion protein comprises two TAA binding regions.

12. The antigen targeting, anti-CD16A, and immune effector cell activating trifunctional fusion protein according to claim 11, wherein the fusion protein comprises a first monomer and a second monomer; the two TAA binding regions are the first TAA binding region and the second binding region, respectively; wherein,
the first monomer comprises: the first TAA binding region and a first Fc chain; the second monomer comprises: the CD16A binding region, the second TAA binding region, the IL-15/IL-15Rα complex and a second Fc chain; the first TAA binding region is a Fab fragment targeting TAA; the CD16A binding region is a Fv fragment targeting CD16A; the second TAA binding region is a Fv fragment targeting TAA;
or,
the first monomer comprises: the first TAA binding region, the CD16A binding region and a first Fc chain; the second monomer comprises: the second TAA binding region, the IL-15/IL-15Rα complex and a second Fc chain; the CD16A binding region is a Fv fragment targeting CD16A; both the first TAA binding region and the second TAA binding region are Fv fragments targeting TAA;
and,
the first Fc chain and the second Fc chain polymerize to form the Fc domain.

13. The antigen targeting, anti-CD16A, and immune effector cell activating trifunctional fusion protein according to claim 12, wherein the first monomer comprises a first peptide chain and a second peptide chain, the second monomer comprises a third peptide chain and a fourth peptide chain;
the first peptide chain comprises: a TAA binding antibody variable region, an antibody constant region;
the second peptide chain comprises: a TAA binding antibody variable region, an antibody constant region; the constant region of the second peptide chain comprises a first Fc chain;
the third peptide chain comprises: a TAA binding antibody variable region, a CD16A antibody variable region, a cytokine, and a second Fc chain;
the fourth peptide chain comprises: a TAA binding antibody variable region, a CD16A antibody variable region, and a cytokine;
the pairing regions of the TAA binding antibody variable region and the antibody constant region of the first peptide chain, as well as the TAA binding antibody variable region and the constant region of the second peptide chain form the first TAA binding region, and the first TAA binding region is a Fab fragment targeting TAA;
the TAA binding antibody variable region of the third peptide chain and the TAA binding antibody variable region of the fourth peptide chain form the second TAA binding region, and the second TAA binding region is a Fv fragment targeting TAA;
the CD16A binding antibody variable region of the third peptide chain and the CD16A binding antibody variable region of the fourth peptide chain form the CD16A binding region, and the CD16A binding region is a Fv fragment targeting CD16A;
the cytokine of the third peptide chain interacts with the cytokine of the fourth peptide chain to form the IL-15/IL-15Rα complex.

14. The antigen targeting, anti-CD16A, and immune effector cell activating trifunctional fusion protein according to claim 13, wherein, from the N-terminus to the C-terminus of the peptide chain, the fusion order of the amino acid fragments of the third peptide chain or the fourth peptide chain comprises any one of the following orders; wherein, "~" represents a connecting fragment:
(1) TAA binding antibody variable region~CD16A binding antibody variable region-cytokine;
(2) TAA binding antibody variable region~cytokine~CD 16A binding antibody variable region;
(3) CD16A binding antibody variable region~TAA binding antibody variable region-cytokine;
(4) CD 16A binding antibody variable region-cytokine-TAA binding antibody variable region;
(5) Cytokine~TAA binding antibody variable region~CD16A binding antibody variable region;
(6) Cytokine~CD16A binding antibody variable region~TAA binding antibody variable region.

15. The antigen targeting, anti-CD16A, and immune effector cell activating trifunctional fusion protein according to claim 12, wherein the fusion protein comprises a first monomer and a second monomer; wherein
the first peptide chain comprises: a TAA binding antibody variable region, a CD16A binding antibody variable region and an antibody constant region;
the second peptide chain comprises: a TAA binding antibody variable region, a CD16A binding antibody variable region, an antibody constant region; the constant region of the second peptide chain comprises a first Fc chain;
the third peptide chain comprises: a TAA antibody variable region, a cytokine, and a second Fc chain;
the fourth peptide chain comprises: a TAA antibody variable region, a cytokine;
the TAA binding antibody variable region of the first peptide chain and the TAA binding antibody variable region of the second peptide chain form the first TAA binding region, and the first TAA binding region is a Fv fragment targeting TAA;
the CD16A binding antibody variable region of the first peptide chain and the CD16A binding antibody variable region of the second peptide chain form the CD16A binding region, and the CD16A binding region is a Fv fragment targeting CD16A;
the TAA binding antibody variable region of the third peptide chain and the TAA binding antibody variable region of the fourth peptide chain form the second TAA binding region, and the second TAA binding region is a Fv fragment targeting TAA;
the cytokine of the third peptide chain interacts with the cytokine of the fourth peptide chain to form the IL-15/IL-15Rα complex.

16. The antigen targeting, anti-CD16A, and immune effector cell activating trifunctional fusion protein according to claim 15, wherein:
in the first peptide chain or the second peptide, from the N-terminus to the C-terminus of the peptide chain, the fusion order of the TAA binding antibody variable region and the CD16A binding antibody variable region is: TAA binding antibody variable region~CD16A binding antibody variable region, or CD16A binding antibody variable region~TAA binding antibody variable region; "~" represents a connecting fragment; or,
in the third peptide chain or the fourth peptide chain, from the N-terminus to the C-terminus of the peptide chain, the fusion order of the TAA binding antibody variable region and the cytokine is: TAA binding antibody variable region~cytokine, or cytokine~TAA binding antibody variable region.

17. The antigen targeting, anti-CD16A, and immune effector cell activating trifunctional fusion protein according to any one of claims 8 or 9, or 13-16, wherein the TAA binding antibody variable region is selected from the VH or VL domain targeting TAA, the CD16A binding antibody variable region is selected from the VH or VL domain targeting CD16A, and the cytokine is selected from IL-15 or IL-15Rα; the antibody constant region comprises a CL domain or a CH1 domain.

18. The antigen targeting, anti-CD16A, and immune effector cell activating trifunctional fusion protein according to claim 17, wherein, there are one or more pairs of disulfide bonds between the VH domain and VL domain, and they comprise any one or more of the following mutation combinations, counted according to EU numbering:
| | **Disulfide bond modification site** | |
|---|---|---|
| | VH | VL |
| Combination 1 | 37C | 95C |
| Combination 2 | 44C | 100C |
| Combination 3 | 44C | 105C |
| Combination 4 | 45C | 87C |
| Combination 5 | 100C | 50C |
| Combination 6 | 100bC | 49C |
| Combination 7 | 98C | 46C |
| Combination 8 | 101C | 46C |
| Combination 9 | 105C | 43C |
| Combination | 106C | 57C |

19. The antigen targeting, anti-CD16A, and immune effector cell activating trifunctional fusion protein according to claim 1, wherein the IL-15 comprises: IL-15 and mutations, truncations, and various derivatives thereof that can bind to IL-15Ra; the IL-15Ra comprises: IL-15Ra and mutations, truncations, and various derivatives thereof that can bind to IL-15; preferably, the IL-15 includes but is not limited to any combination of the following mutation methods, with the counting method starting to count the first amino acid of the IL-15 sequence as the first position;
| Combinations | **IL-15 mutations** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |
| 21 | D30N/E64Q/N65D |
;
or, the IL-15/IL-15Ra complex includes but is not limited to any combination of the following mutation methods, with the counting method starting to count the first amino acid of the IL-15 or IL-15Ra sequence as the first position;
| **Combinations** | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C. |

20. The antigen targeting, anti-CD16A, and immune effector cell activating trifunctional fusion protein according to claim 1, wherein the TAAis selected from CD20, CD19, CD30, CD33, CD38, CD40, CD52, slamf7, GD2, CD24, CD47, CD133, CD217, CD239, CD274, CD276, CEA, Epcam, Trop2, TAG72, MUC1, MUC16, mesothelin, folr1, CLDN18.2, EGFR, EGFR VIII, C-MET, HER2, FGFR2, FGFR3, PSMA, PSCA, EphA2, ADAM17, 17-A1, NKG2D ligands, MCSP, LGR5, SSEA3, SLC34A2, BCMA, GPNMB, CCR4, VEGFR-2, CD6, integrin α4, PDGFRα, NeuGcGM3, integrin αVβ3, CD51, CTAA16.88, CD22, ROR1, CSPG4, SS1 or IGFR1.

21. The antigen targeting, anti-CD16A, and immune effector cell activating trifunctional fusion protein according to claim 1, wherein the Fc domain is selected from Human IgG1 Fc, Human IgG2 Fc, Human IgG3 Fc, Human IgG4 Fc or variants thereof, preferably IgG1 Fc, or Human IgG4 Fc or variants thereof; the Fc domain is in the form of a Fc heterodimer; preferably, the Fc heterodimer includes but is not limited to a combination of the following mutations, which are counted according to EU numbering:
| Combinations | FC | Heterodimer mutations (Eu numbering) |
|---|---|---|
| 1 | The first FC chain | T366Y |
| | The second FC chain | Y407T |
| 2 | The first FC chain | T366W |
| | The second FC chain | T366S/L368A/Y407V |
| 3 | The first FC chain | S354C/T366W |
| | The second FC chain | Y349C/T366S/L368A/Y407V |
| 4 | The first FC chain | S364H/F405A |
| | The second FC chain | Y349T/T394F |
| 5 | The first FC chain | T350V/L351Y/F405A/Y407V |
| | The second FC chain | T350V/T366L/K392L/T394W |
| 6 | The first FC chain | K392D/K409D |
| | The second FC chain | E356K/D399K |
| 7 | The first FC chain | D221E/P228E/L368E |
| | The second FC chain | D221R/P228R/K409R |
| 8 | The first FC chain | K360E/K409W |
| | The second FC chain | Q347R/D399V/F405T |
| 9 | The first FC chain | K360E/K409W/Y349C |
| | The second FC chain | Q347R/D399V/F405T/S354C |
| 10 | The first FC chain | K370E/K409W |
| | The second FC chain | E357N/D399V/F405T |
| 11 | The first FC chain | F405L |
| | The second FC chain | K409R |
| 12 | The first FC chain | K360D/D399M/Y407A |
| | The second FC chain | E345R/Q347R/T366V/K409V |
| 13 | The first FC chain | Y349S/K370Y/T366M/K409V |
| | The second FC chain | E356G/E357D/S364Q/Y407A |
| 14 | The first FC chain | L351D/L368E |
| | The second FC chain | L351K/T366K |
| 15 | The first FC chain | GQPFRPEVHLLPPSREEMTKNQVSLTCLARGFYPK DIAVEWESNGQPENNYKTTPSRQEPSQGTTTFAVT SKLTVDKSRWQQGNVFSCSVMHEALHNHYTQKTI SL |
| | The second FC chain | GQPREPQVYTLPPPSEELALNELVTLTCLVKGFYP SDIAVEWLQGSQELPREKYLTWAPVLDSDGSFFLY SILRVAAEDWKKGDTFSCSVMHEALHNHYTQKSLD R |
| 16 | The first FC chain | L368D/K370S |
| | The second FC chain | E357Q/S364K |
| 17 | The first FC chain | S354C/T366W/**K409A** |
| | The second FC chain | Y349C/T366S/L368A/Y407V/**F405K** |
| 18 | The first FC chain | S354C/T366W/**F405K/K360EQ347E** |
| | The second FC chain | Y349C/T366S/L368A/Y407V/**Q347R/T394W** |
| 19 | The first FC chain | T366W/**K409A** |
| | The second FC chain | T366S/**L368G/Y407A/F405K** |
| 20 | The first FC chain | knobs(T366W/**F405K**) |
| | The second FC chain | holes(T366S/**L368G/Y407A/K409A**) |
| 21 | The first FC chain | Q347A/S364K/T366V/K370T/K392Y/F405S/Y407V/K 409W/T411N |
| | The second FC chain | Q347E/Y349A/L351F/S364T/T366V//K370T/T394D/V 397L/D399E/D401Q/F405A/Y407S/K409R/T411R |
| 22 | The first FC chain | K274Q/N276K/Y300F/A339T/Q347A/S364K/T366V/ K370T/N384S/K392Y/V397M/F405S/Y407V/K409W/ T411N/V422I/H435R/Y436F |
| | The second FC chain | Q347E/Y349A/L351F/S364T/T366V//K370T/T394D/V 397L/D399E/D401Q/F405A/Y407S/K409R/T411R |

22. The antigen targeting, anti-CD 16A, and immune effector cell activating trifunctional fusion protein according to claim 1, wherein the Fc domain selectively eliminates immune effector functions, preferably comprises any one of the following mutation modes, which are counted according to EU numbering:
| **IgG** | FC MutatefEU numbing) |
|---|---|
| **IgG1** | L234A,L235A |
| | L234A,L235A,P329G |
| | L234F,L235E.P331S |
| | D265A,N297A |
| | L234F,L235E,N297A |
| | L234F,L235E,D265A |
| | L234A,L235E,P331S |
| | L234A,L235E,N297A |
| | L234A,L235E,D265A |
| | L234A,L235,P331S |
| | L234A,L235A,N297A |
| | L234A,L235A,D265A |
| | L235E,D265A,P331S |
| | L235E,N297A,P331S |
| | L235E,N297A |
| | L235A,D265A,P331S |
| | L235A,N297A,P331S |
| | N297Q |
| | N297A |
| | N297G |
| | A287C, N297G, L306C |
| | R292C, N297G, V302C |
| **hIgG4** | S228P,L235E,P329G |
| | S228P,L235E |
| | S228P,F234A,L235E |
| | S228P,F234A,L235A |
| **IgG2m4** | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCVV VDVSQEDPEVQFNWYVDGVEVHNAKTKPREEQFNSTYRVVSVLTV LHQDWLNGKEYKCKVSNKGLPSSIEKTISKAKGQPREPQVYTLPPSQ EEMTKNQVSLTCLVKGFYPSDIAVEWESNGQPENNYKTTPPVLDSD GSFFLYSRLTVDKSRWQEGNVFSCSVMHEALHNHYTQKSLSLSLGK |
| | N297A |
| | N297Q |
| | N297G |

23. The antigen targeting, anti-CD16A, and immune effector cell activating trifunctional fusion protein according to claims 7 or 12, wherein the first Fc chain does not bind to protein A, preferably comprises mutations H435R or H435R/Y436F, according to EU numbering; the second Fc chain can bind to protein A.

24. The antigen targeting, anti-CD16A, and immune effector cell activating trifunctional fusion protein according to claim 1, wherein the CD16A binding region comprises a VH domain shown in SEQ ID NO: 97 and a VL domain shown in SEQ ID NO: 96; alternatively, the sequence of IL-15 is shown in SEQ ID NO: 104 or 105; alternatively, the sequence of IL-15Ra may be represented by any one of SEQ ID NOs: 106 to 112; preferably, there is at least one pair of disulfide bond between VH and VL in the CD16A binding region, and/or between IL-15 and IL-15Ra.

25. The antigen targeting, anti-CD16A, and immune effector cell activating trifunctional fusion protein according to claim 1, wherein the antigen targeting, anti-CD16A, and immune effector cell activating trifunctional fusion protein is obtained by fusing amino acid fragments shown in any one of the following sets of sequences or amino acid fragments with over 90% identity:
(1) SEQ ID NO: 01, 02, 03, 04;
(2) SEQ ID NO: 01, 05, 03, 06;
(3) SEQ ID NO: 07, 08, 09, 10;
(4) SEQ ID NO: 01, 02, 11, 12;
(5) SEQ ID NO: 01, 02, 13, 14;
(6) SEQ ID NO: 01, 05, 11, 15;
(7) SEQ ID NO: 01, 05, 13, 16;
(8) SEQ ID NO: 38, 39, 40, 41;
(9) SEQ ID NO: 38, 39, 42, 43;
(10) SEQ ID NO: 38, 39, 44, 45;
(11) SEQ ID NO: 38, 39, 46, 47;
(12) SEQ ID NO: 48, 49, 50, 51;
(13) SEQ ID NO: 48, 49, 52, 53;
(14) SEQ ID NO: 48, 49, 54, 55;
(15) SEQ ID NO: 48, 49, 56, 57;
(16) SEQ ID NO: 58, 59, 60, 61;
(17) SEQ ID NO: 58, 59, 62, 63;
(18) SEQ ID NO: 58, 59, 64, 65;
(19) SEQ ID NO: 58, 59, 66, 67;
(20) SEQ ID NO: 68, 69, 70, 71;
(21) SEQ ID NO: 72, 73, 70, 71;
(22) SEQ ID NO: 74, 75, 70, 71;
(23) SEQ ID NO: 76, 77, 70, 71;
(24) SEQ ID NO: 78, 79, 70, 71;
(25) SEQ ID NO: 38, 39, 80, 81;
(26) SEQ ID NO: 38, 39, 82, 83;
(27) SEQ ID NO: 38, 39, 84, 85;
(28) SEQ ID NO: 38, 39, 86, 87;
(29) SEQ ID NO: 38, 39, 88, 89;
(30) SEQ ID NO: 38, 39, 90, 91;
(31) SEQ ID NO: 38, 39, 92, 93;
(32) SEQ ID NO: 38, 39, 94, 95.

26. The antigen targeting, anti-CD16A, and immune effector cell activating trifunctional fusion protein according to claim 1, wherein the antigen further comprises infectious disease related antigens, pathogens, and immune function related antigens.

27. A nucleic acid molecule that encodes the antigen targeting, anti-CD16A, and immune effector cell activating trifunctional fusion protein according to any one of claims 1-26.

28. A pharmaceutical composition, which comprises the antigen targeting, anti-CD16A, and immune effector cell activating trifunctional fusion protein according to any one of claims 1-26, and pharmaceutically acceptable carriers.

29. Use of the antigen targeting, anti-CD16A, and immune effector cell activating trifunctional fusion protein according to any one of claims 1-26 in the preparation of drugs for inhibiting or treating cancer, infection, and immunomodulatory diseases.

30. The use according to claim 29, wherein the cancer comprises prostate cancer, lung cancer, colon cancer, rectal cancer, bladder cancer, melanoma, kidney cancer, oral cancer, pharyngeal cancer, pancreatic cancer, uterine cancer, thyroid cancer, skin cancer, head and neck cancer, cervical cancer, ovarian cancer or hematological system cancer.

31. A protein complex unit, which comprises: the CD16A antibody variable region and the cytokine of the third peptide chain, the CD16A antibody variable region and the cytokine of the fourth peptide chain in the antigen targeting, anti-CD16A, and immune effector cell activating trifunctional fusion protein according to claim 8, and several connecting fragments;
the CD16A antibody variable region of the third peptide chain is fused with the cytokine of the third peptide chain through a connecting fragment; the CD16A antibody variable region of the fourth peptide chain is fused with the cytokine of the fourth peptide chain through a connecting fragment;
the CD16A antibody variable region of the third peptide chain and the CD16A antibody variable region of the fourth peptide chain are selected from the VH domain or VL domain targeting CD16A, and pair to form a Fv fragment;
the cytokine of the third peptide chain and the cytokine of the fourth peptide chain are selected from IL 15 or IL15Rα, and interact to form an IL15/IL15Rα complex;
preferably, there are more than one pair of disulfide bonds between the VH domain and the VL domain, and/or between the IL15 and IL15Rα.

32. A nucleic acid molecule encoding the protein complex unit according to claim 31.
